# EUROPÄISCHE PATENTSCHRIFT

(11) **EP 2 606 936 B1**
(45) Veröffentlichungstag und Bekanntmachung des Hinweises auf die Patenterteilung: **07.06.2017**
(21) Anmeldenummer: 12191940.1
(22) Anmeldetag: 09.11.2012
(51) Int. Cl.: A61Q 5/12, A61K 8/06, A61K 8/37, A61K 8/86, A61K 8/64, A61K 8/65, A61K 8/67, A61K 8/60, A61K 8/49, A61K 8/34, A61K 8/44, A61K 8/92, A61K 8/41, A61K 8/73, A61K 8/81

(54) **Verfahren zur Haarbehandlung mit Haarkur enthaltend einen speziellen Emulgator und Proteinhydrolysate**
Process for hair treatment with hair conditioner comprising a special emulsifier and protein hydrolysates
Procédé de soin capillaire avec un produit de soin comprenant un émulsifiant spécial et des hydrolysats protéiques

(30) Priorität: 19.12.2011 DE 102011088929
(43) Veröffentlichungstag der Anmeldung: 26.06.2013
(73) Patentinhaber: Henkel AG & Co. KGaA, 40589 Düsseldorf (DE)
(72) Erfinder: Battermann, Marlene, 21271 Asendorf (DE); Müller-von Borstel, Christin, 21682 Stade (DE)

(56) Entgegenhaltungen:
- EP-A1- 1 133 973
- DE-A1- 4 009 617
- DE-A1- 10 048 366
- US-A1- 2002 035 046
- M KANOUNI ET AL: "Preparation of a stable double emulsion (W1/O/W2): role of the interfacial films on the stability of the system", ADVANCES IN COLLOID AND INTERFACE SCIENCE, Bd. 99, Nr. 3, 1. Dezember 2002 (2002-12-01), Seiten 229-254, XP055115976, ISSN: 0001-8686, DOI: 10.1016/S0001-8686(02)00079-9
- Croda: "Cithrol (TM) DPHS", US Internet Archive Wayback Machine, 4. Juni 2011 (2011-06-04), Seiten 1-1, XP002724220, Gefunden im Internet: URL:https://web.archive.org/web/2011060410 5839/http://www.croda.com/home.aspx?view=d tl&d=content&s=149&r=346&p=2204&productnam e=cithrol%20dphs&chemicaldescription=&chem icalgroup=&function=&application=&prodID=7 01&sumID=941 [gefunden am 2014-04-30]
- NN: "W/O emulsions improved for formulators and consumers", Personal Care Ingredients Formulation Manufacture, 1. Januar 2011 (2011-01-01), 1. Januar 2011 (2011-01-01), Seiten 1-1, XP002724221, Gefunden im Internet: URL:http://www.personalcaremagazine.com/Pr int.aspx?Story=7736 [gefunden am 2014-05-12]
- "Water in oil emulsions comprising a high level content of active agents", IP.COM JOURNAL, IP.COM INC., WEST HENRIETTA, NY, US, 25. Mai 2010 (2010-05-25), XP013138358, ISSN: 1533-0001

## Beschreibung

Die Erfindung betrifft Haarbehandlungsmittel enthaltend PEG-30 Dipolyhydroxystearate und Proteinhydrolysaten sowie die Verwendung dieser Mittel zur Haarbehandlung.

Nicht zuletzt durch die starke Beanspruchung der Haare, beispielsweise durch das Färben oder Dauerwellen als auch durch die Reinigung der Haare mit Shampoos und durch Umweltbelastungen, nimmt die Bedeutung von Pflegeprodukten mit möglichst langanhaltender Wirkung zu.

Die bekannten Wirkstoffe können jedoch nicht alle Bedürfnisse in ausreichendem Maße abdecken. Zudem liegen die konventionellen Haarbehandlungsmittel in Form einer O/W - Emulsion vor. EP 1133973 A1 offenbart Haarpflegeemulsionen in Form von W/O-Emulsionen. Es besteht daher weiterhin ein Bedarf nach Wirkstoffen bzw. Wirkstoffkombinationen für kosmetische Mittel mit guten pflegenden Eigenschaften. Gesucht sind daher weiterhin stabile Systeme mit verbesserten Pflegeeigenschaften.

Überraschenderweise wurde nun jedoch gefunden, dass Haarbehandlungsmittel enthaltend PEG-30 Dipolyhydroxystearate und Proteinhydrolysate, insbesondere bei weiterer Anwesenheit ausgewählter quaternärer Verbindungen zu stabilen wirksamen Zusammensetzungen führen und sich als W/O - Emulsionen formulieren lassen.

Gleichzeitig führt die Verwendung dieser Kombinationen zu überraschend guten Eigenschaften der behandelten keratinischen Fasern, insbesondere zu einem deutlich verbesserten Glanz und einer gesteigerten Haltbarkeit der Pflegeleistung und des Glanzes über mehrere Haarwäschen hinweg. Die gesteigerten Pflegeleistungen umfassen verbesserte Kämmbarkeiten, eine verbesserte Haarstruktur, eine verbesserte Elastizität als auch eine deutlich gesteigerte Waschbeständigkeit gefärbten Haares, sowie eine längere Haltbarkeit bei einer gleichzeitigen besseren Umformleistung bei Wellvorgängen wie Wasserwelle und Dauerwelle.

Diese positiven Eigenschaften werden sowohl bei einer typischen rinse-off als auch bei einer leave-on Applikation erhalten. In hervorragender Weise eignet sich die erfindungsgemäße Zusammensetzung neben den klassischen Cremeformulierungen auch für die Sprühapplikation sowohl als non-Aerosol wie auch als Aerosol. Hierbei wird gegenüber den bislang üblichen Produkten ein außerordentlich gleichmäßiges Sprühbild erzielt. Dadurch wird die gesamte Zusammensetzung sehr gleichmäßig auf dem Haar verteilt.

Ein erster Gegenstand der vorliegenden Erfindung ist daher eine Zusammensetzung, welche in einem kosmetischen Träger PEG-30 Dipolyhydroxystearate zur Behandlung von keratinischen Fasern, enthaltend -jeweils bezogen auf die gesamte Zusammensetzung -
a) mindestens PEG-30 Dipolyhydroxystearate in einer Gesamtmenge von 0,01 bis 5,0 Gew.% und
b) mindestens ein Proteinhydroylsat sowie Mischungen von Proteinhydrolysaten in einer Gesamtmenge von 0,01 bis 10,0 Gew.%
c) in Form einer W/O Emulsion mit einem Wasseranteil von mindestens 50 Gew.%.

Die Inhaltsstoffe a) und b) werden den nachfolgend detailliert beschrieben. Soweit nachstehend vom Wirkstoffkomplex (A) gesprochen wird, bezieht sich diese Aussage auf die in den erfindungsgemäßen Mitteln zwingend enthaltenen Inhaltsstoffe a) und b).

Haarbehandlungsmittel im Sinne der vorliegenden Erfindung sind beispielsweise Haarkonditionierer, Haarsprays, Haarspülungen, Haarkuren, Haarpackungen, Haar-Tonics, Haarfestiger, Haarlegemittel, Haarstyling-Zubereitungen, Fönwell-Lotionen, Schaumfestiger, Haargele, Haarwachse oder deren Kombinationen. Bevorzugte erfindungsgemäße Mittel sind Konditioniermittel oder Haar-Tonics. Die erfindungsgemäßen Mittel liegen als W/O - Emulsion vor. Unter Kämmbarkeit versteht sich erfindungsgemäß sowohl die Kämmbarkeit der nassen Faser, als auch die Kämmbarkeit der trockenen Faser. Als Maß für die Kämmbarkeit dient die aufgewendete Kämmarbeit oder die aufgewendete Kraft während des Kämmvorganges eines Faserkollektivs. Die Meßparameter können durch den Fachmann sensorisch beurteilt oder durch Messeinrichtungen quantifiziert werden.

Als Griff definiert sich die Taktilität eines Faserkollektivs, wobei der Fachmann sensorisch die Parameter Fülle und Geschmeidigkeit des Kollektivs fühlt und bewertet.

Unter Formgebung wird die Fähigkeit verstanden, einem Kollektiv zuvor behandelter keratinhaltiger Fasern, insbesondere menschlicher Haare, eine Formänderung zu verleihen. In der Haarkosmetik wird auch von Frisierbarkeit gesprochen.

Der kosmetische Träger c) ist erfindungsgemäß eine W/O - oder W/O/W - Emulsionen in Form von Cremes oder Gelen oder andere Zubereitungen, die insbesondere für die Anwendung auf dem Haar geeignet sind. Die kosmetischen Träger können insbesondere wässrig oder wässrigalkoholisch sein.

Ein wässriger kosmetischer Träger enthält mindestens 50 Gew.-% Wasser.

Unter wässrig-alkoholischen kosmetischen Trägern sind im Sinne der vorliegenden Erfindung wässrige Lösungen enthaltend 3 bis 70 Gew.-% eines C₁-C₆-Alkohols, insbesondere Methanol, Ethanol bzw. Propanol, Isopropanol, Butanol, Isobutanol, tert.-Butanol, n-Pentanol, iso-Pentanole, n-Hexanol, iso-Hexanole, Glykol, Glycerin, 1,2-Pentandiol, 1,5-Pentandiol, 1,2-Hexandiol oder 1,6-Hexandiol zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Der Inhaltstoff a) ist PEG-30 Dipolyhydroxystearate. Diese Verbindung wird in den kosmetischen Zusammensetzungen in einer Gesamtmenge von 0,01 bis 5,0 Gew.%, vorzugsweise von 0,01 bis 4,0 Gew.%, besonders bevorzugt von 0,05 bis 3,5 Gew% und höchst bevorzugt 0,05 bis 3,0 Gew.%, jeweils bezogen auf die gesamte Zusammensetzung verwendet.

Der zweite wesentliche Inhaltstoff b) in dem erfindungsgemäßen Wirkstoffkomplex sind Proteinhydrolysate und/oder dessen Derivate.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Weiterhin sind erfindungsgemäß bevorzugte pflanzliche Proteinhydrolysaten wie beispielsweise Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Weitere erfindungsgemäß bevorzugte Proteinhydrolysate sind maritimen Ursprunges. Hierzu zählen beispielsweise Kollagenhydrolysate von Fischen oder Algen sowie Proteinhydrolysate von Muscheln bzw. Perlenhydrolysate. Beispiele für erfindungsgemäße Perlenextrakte sind die Handelsprodukte Pearl Protein Extract BG^{®} oder Crodarom^{®} Pearl.

Am bevorzugtesten sind Proteinhydrolysate auf der Basis von Kollagen, Seide, Keratin, Milcheiweiß, Soja, Mandeln, Weizen, Fischen, Algen oder Perlen. Höchst bevorzugt sind die Hydrolysate auf der Basis von Seide, Keratin, Milcheiweiß, Fischen, Algen oder Perlen. Ein Beispiel für ein erfindungsgemäß besonders geeignetes Proteinhydrolysat ist unter der Handelsbezeichnung ProSina® von der Fa. Croda erhältlich.

Die Proteinhydrolysate sind in den Zusammensetzungen in Konzentrationen von 0,001 Gew.% bis zu 10 Gew.%, vorzugsweise von 0,001 Gew.% bis zu 7,5 Gew.%, ganz besonders bevorzugt in Mengen von 0,01 Gew.% bis zu 5 Gew.%, höchst bevorzugt in Mengen von 0,01 bis zu 3,0 Gew.% und allerhöchst bevorzugt in Mengen von 0,05 bis 2,0 Gew.% enthalten.

In einer zweiten bevorzugten Ausführungsform der vorliegenden Erfindung wird die Wirkung durch die Anwesenheit einer ausgewählten Verbindung mit einer quarternären Ammoniumgruppe weiter gesteigert.

Quaternäre Ammoniumverbinungen sind prinzipiell monomere kationische oder amphotäre Ammoniumverbindungen, monomere Amine, Aminoamide, polymere kationische Ammoniumverbindungen sowie polymere amphotere Ammoniumverbindungen. Aus dieser Vielzahl an möglichen quaternären Ammoniumverbindungen haben sich die folgenden Gruppen als besonders geeignet erwiesen und werden jeweils für sich genommen in einer Menge von 0,1 bis 10,0 Gew.% eingesetzt. Diese Menge wird auch nicht unter- oder überschritten, wenn eine Mischung unterschiedlicher Verbindungen der quaternären Ammoniumverbindungen verwendet wird.

Esterquats gemäß der Formel (Tkat1-2) bilden die erste Gruppe.

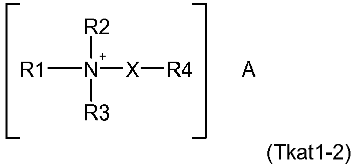

Hierin sind die Reste R1, R2 und R3 jeweils unabhängig voneinander und können gleich oder verschieden sein. Die Reste R1, R2 und R3 bedeuten:
- ein verzweigter oder unverzweigter Alkylrest mit 1 bis 4 Kohlenstoffatomen, welcher mindestens eine Hydroxylgruppe enthalten kann, oder
- ein gesättigter oder ungesättigter, verzweigter oder unverzweigter oder ein cyclischer gesättigter oder ungesättigter Alkylrest mit 6 bis 30 Kohlenstoffatomen, welcher mindestens eine Hydroxylgruppe enthalten kann, oder
- ein Aryl oder Alkarylrest, beispielsweise Phenyl oder Benzyl,
- den Rest (- X - R4), mit der Maßgabe, dass höchstens 2 der Reste R1, R2 oder R3 für diesen Rest stehen können:
   Der Rest -(X - R4) ist mindestens 1 bis 3 mal enthalten.

Hierin steht X für:
1) -(CH2)n- mit n = 1 bis 20, vorzugsweise n = 1 bis 10 und besonders bevorzugt n = 1 - 5, oder
2) -(CH2-CHR5-O)n- mit n = 1 bis 200, vorzugsweise 1 bis 100, besonders bevorzugt 1 bis 50, und besonders bevorzugt 1 bis 20 mit R5 in der Bedeutung von Wasserstoff, Methyl oder Ethyl,
3) eine Hydroxyalkylgruppe mit ein bis vier Kohlenstoffatomen, welche verzweigt oder unverzweigt sein kann, und welche mindestens eine und höchstens 3 Hydroxygruppen enthält. Beispiele sind: -CH₂OH, -CH₂CH₂OH, -CHOHCHOH, -CH₂CHOHCH₃, -CH(CH₂OH)₂, -COH(CH₂OH)₂, -CH₂CHOHCH₂OH, -CH₂CH₂CH₂OH und Hydroxybutylreste,
und R4 steht für:
1) R6-O-CO-, worin R6 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, oder
2) R7-CO-, worin R7 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann,
und A steht für ein physiologisch verträgliches organisches oder anorganisches Anion und wird an dieser Stelle stellvertretend für alle auch im folgenden beschriebenen Strukturen definiert. Das Anion aller beschriebenen kationischen Verbindungen ist ausgewählt aus den Halogenidionen, Fluorid, Chlorid, Bromid, lodid, Sulfaten der allgemeinen Formel RSO₃⁻, worin R die Bedeutung von gesättigtem oder ungesättigtem Alkylresten mit 1 bis 4 Kohlenstoffatomen hat, oder anionischen Reste organischer Säuren wie Maleat, Fumarat, Oxalat, Tartrat, Citrat, Lactat oder Acetat.

Solche Produkte werden beispielsweise unter den Warenzeichen Rewoquat^{®}, Stepantex^{®}, Dehyquart^{®}, Armocare^{®} und Akypoquat^{®} vertrieben. Die Produkte Armocare^{®} VGH-70, Dehyquart^{®} F-75, Dehyquart^{®} C-4046, Dehyquart^{®} L80, Dehyquart^{®} F-30, Dehyquart^{®} AU-35, Rewoquat^{®} WE18, Rewoquat^{®} WE38 DPG, Stepantex^{®} VS 90 und Akypoquat^{®} 131 sind Beispiele für diese Esterquats.

Weitere erfindungsgemäß besonders bevorzugte Verbindungen der Formel (Tkat1-2) zählen zur Formel (Tkat1-2.1), den kationischen Betainestern. 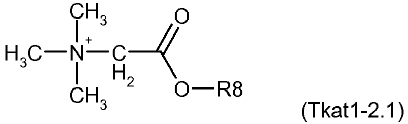 R8 entspricht in seiner Bedeutung R7.

Besonders bevorzugt sind die Esterquats mit den Handelsbezeichnungen Armocare^{®} VGH-70, sowie Dehyquart^{®} F-75, Dehyquart^{®} L80, Stepantex^{®} VS 90 und Akypoquat^{®} 131.

Eine weitere Gruppe sind quartäre Imidazolinverbindungen. Die im Folgenden dargestellte Formel (Tkat2) zeigt die Struktur dieser Verbindungen.

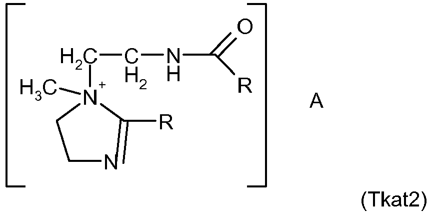

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel (Tkat2) enthalten für R jeweils den gleichen Kohlenwasserstoffrest. Die Kettenlänge der Reste R beträgt bevorzugt 12 bis 21 Kohlenstoffatome. A steht für ein Anion wie zuvor beschrieben. Besonders erfindungsgemäße Beispiele sind beispielsweise unter den INCII - Bezeichnungen Quaternium-27, Quaternium-72, Quaternium-83 und Quaternium-91 erhältlich. Höchst bevorzugt ist erfindungsgemäß Quaternium-91.

In einer besonders bevorzugten Ausführungsform der Erfindung enthalten die erfindungsgemäßen Mittel weiterhin mindestens ein Amin und/oder kationisiertes Amin, insbesondere ein Amidoamin und/oder ein kationisiertes Amidoamin mit den folgenden Strukturformeln:

R1 - NH - (CH₂)ₙ - N+R²R³R⁴ A (Tkat3)

worin R1 ein Acyl-oder Alkylrest mit 6 bis 30 C-Atomen, welche verzweigt oder unverzweigt, gesättigt oder ungesättigt sein können, und wobei der Acylrest und/oder der Alkylrest mindestens eine OH-Gruppe enthalten können, und
R2, R3 und R4 jeweils unabhängig voneinander
   1) Wasserstoff oder
   2) ein Alkylrest mit 1 bis 4 C-Atomen, welcher gleich oder verschieden, gesättigt oder ungesättigt sein kann, und
   3) eine verzweigte oder unverzweigte Hydroxyalkylgruppe mit ein bis 4 Kohlenstoffatomen mit mindestens einer und höchstens drei Hydroxygruppen beispielsweise -CH₂OH, -CH₂CH₂OH, -CHOHCHOH, -CH₂CHOHCH₃, -CH(CH₂OH)₂, -COH(CH₂OH)₂, -CH₂CHOHCH₂OH, -CH₂CH₂CH₂OH und Hydroxybutylreste, und
A ein Anion wie zuvor beschrieben und
n eine ganze Zahl zwischen 1 und 10 bedeuten.

Bevorzugt wird eine Zusammensetzung, in welcher das Amin und/oder das quaternisierte Amin gemäß allgemeiner Formeln (Tkat3) ein Amidoamin und/oder ein quaternisiertes Amidoamin ist, worin R1 ein verzweigter oder unverzweigter, gesättigter oder ungesättigter Acylrest mit 6 bis 30 C-Atomen, welcher mindestens eine OH-Gruppe enthalten kann, bedeutet. Bevorzugt ist hierbei ein Fettsäurerest aus Ölen und Wachsen, insbesondere aus natürlichen Ölen und Wachsen. Als Beispiele hierfür kommen Lanolin, Bienen- oder Candellilawachse in Betracht.

Bevorzugt sind auch solche Amidoamine und/oder quaternisierte Amidoamine, in denen R2, R3 und/oder R4 in der Formel (Tkat3) ein Rest gemäß der allgemeinen Formel CH₂CH₂OR5 bedeuten, worin R5 die Bedeutung von Alkylresten mit 1 bis 4 Kohlenstoffatomen, Hydroxyethyl oder Wasserstoff haben kann. Die bevorzugte Größe von n in der allgemeinen Formel (Tkat8) ist eine ganze Zahl zwischen 2 und 5.

Die Alkylamidoamine können sowohl als solche vorliegen und durch Protonierung in entsprechend saurer Lösung in eine quaternäre Verbindung in der Zusammensetzung überführt werden. Erfindungsgemäß bevorzugt sind die kationischen Alkylamidoamine.

Beispiele für derartige erfindungsgemäße Handelsprodukte sind Witcamine^{®} 100, Incromine^{®} BB, Mackine^{®} 401 und andere Mackine^{®} -Typen, Adogen^{®} S18V, und als permanent kationische Aminoamine: Rewoquat^{®} RTM 50, Empigen^{®} CSC, Swanol^{®} Lanoquat DES-50, Rewoquat^{®} UTM 50, Schercoquat^{®} BAS, Lexquat^{®} AMG-BEO, oder Incroquat^{®} Behenyl HE.

Weitere quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Behenyltrimethylammoniumchlorid, Distearyldimethylammoniumchlorid, Lauryldimethylammoniumchlorid, Lauryldimethylbenzylammoniumchlorid und Tricetylmethylammoniumchlorid. Die langen Alkylketten der oben genannten Tenside weisen bevorzugt 10 bis 22 Kohlenstoffatome auf. Die zuvor genannten kationischen Tenside können einzeln oder in beliebigen Kombinationen miteinander verwendet werden, wobei Mengen zwischen 0,01 bis 10 Gew.%, bevorzugt in Mengen von 0,01 bis 7,5 Gew.% und ganz besonders bevorzugt in Mengen von 0,1 bis 5,0 Gew.% enthalten. Die allerbesten Ergebnisse werden dabei mit Mengen von 0,1 bis 3,0 Gew.% jeweils bezogen auf die Gesamtzusammensetzung des jeweiligen Mittels erhalten.

Weitere quarternäre Ammoniumverbindungen sind kationische und amphotere Polymer.

Die kationischen und/oder amphoteren Polymere können Homo- oder Copolymere oder Polymere auf Basis natürlicher Polymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid; Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Aus der Vielzahl dieser Polymere haben sich als besonders wirkungsvolle Bestandteile des erfindungsgemäßen Wirkstoffkomplexes erwiesen:
Homopolymere der allgemeinen Formel

-{CH₂-[CR¹COO-(CH₂)ₘN⁺R²R³R⁴]}ₙ X⁻,

in der R¹= -H oder -CH₃ ist, R², R³ und R⁴ unabhängig voneinander ausgewählt sind aus C1-4-Alkyl-, -Alkenyl- oder-Hydroxyalkylgruppen, m = 1, 2, 3 oder 4, n eine natürliche Zahl und X⁻ ein physiologisch verträgliches organisches oder anorganisches Anion ist. Im Rahmen dieser Polymere sind diejenigen erfindungsgemäß bevorzugt, für die mindestens eine der folgenden Bedingungen gilt: R¹ steht für eine Methylgruppe, R², R³ und R⁴ stehen für Methylgruppen, m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Methosulfate und Halogenidionen, insbesondere Chlorid.

Geeignete kationische Polymere, die von synthetischen Poylmeren abgeleitet sind, sind beispielsweise Copolymere A aus A1) 0,1 bis 50 %, vorzugsweise 10 bis 50 % (bezogen auf die Gesamt-Anzahl an Monomeren im Copolymer) Monomeren der Formel (la) 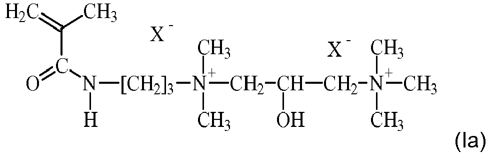 in der X steht für Chlorid, Sulfat, Methosulfat, und A2) Monomere aus der Gruppe Acrylsäure, Methacrylsäure sowie den Alkalimetall- und Ammoniumsalzen dieser Säuren, wobei das Monomere A2 50 bis 99,9 %, vorzugsweise 50 bis 90 % (bezogen auf die Gesamt-Anzahl an Monomeren im Copolymer) des Copolymers ausmacht; enthalten.

Ein höchst bevorzugtes Polymer, welches wie zuvor dargestellt aufgebaut ist, ist unter der Bezeichnung Polyquaternium-74 im Handel erhältlich.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare^{®} CTH (Cosmetic Rheologies) und Synthalen^{®} CR (3V Sigma) im Handel erhältlich.

Das Homopolymer wird bevorzugt in Form einer nichtwäßrigen Polymerdispersion eingesetzt. Solche Polymerdispersionen sind unter den Bezeichnungen Salcare^{®} SC 95 und Salcare^{®} SC 96 im Handel erhältlich.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel

G-O-B-N+RₐR_{b}R_{c} A⁻

G ist ein Anhydroglucoserest, beispielsweise Stärke- oder Celluloseanhydroglucose;
B ist eine divalente Verbindungsgruppe, beispielsweise Alkylen, Oxyalkylen, Polyoxyalkylen oder Hydroxyalkylen;
Rₐ, R_{b} und R_{c} sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in Rₐ, R_{b} und R_{c} vorzugsweise maximal 20 ist;
A⁻ ist ein übliches Gegenanion und ist vorzugsweise Chlorid.

Kationische, also quaternisierte Cellulosen sind mit unterschiedlichem Substitutionsgrad, kationischer Ladungsdichte, Stickstoffgehalt und Molekulargewichten auf dem Markt erhältlich. Beispielsweise wird Polyquaternium-67 im Handel unter den Bezeichnungen Polymer^{®} SL oder Polymer^{®} SK (Amerchol) angeboten. Unter der Handelsbezeichnung Mirustyle^{®} CP der Fa. Croda wird eine weitere höchst bevorzugte Cellulose angeboten. Diese ist eine Trimonium and Cocodimonium Hydroxyethylcellulose als derivatisierte Cellulose mit der INCI-Bezeichnung Polyquaternium-72. Polyquaternium-72 kann sowohl in fester Form als auch bereits in wässriger Lösung vorgelöst verwendet werden.

Weitere kationische Cellulosen sind unter den Bezeichnungen Polymer JR^{®} 400 (Amerchol, INCI-Bezeichnung Polyquaternium-10) sowie Polymer Quatrisoft^{®} LM-200 (Amerchol, INCI-Bezeichnung Polyquaternium-24). Weitere Handelsprodukte sind die Verbindungen Celquat^{®} H 100 und Celquat^{®} L 200. Schließlich liegt unter der Handelsbezeichnung Mirustyle^{®} CP der Fa. Croda mit Trimonium and Cocodimonium Hydroxyethylcellulose eine weitere derivatisierte Cellulose mit der INCI-Bezeichnung Polyquaternium-72 vor. Polyquaternium-72 kann sowohl in fester Form als auch bereits in wässriger Lösung vorgelöst verwendet werden. Besonders bevorzugte kationische Cellulosen sind Polyquaternium-10, Polyquaternium-24, Polyquaternium-67 und Polyquaternium-72.

Geeignete kationische Guarderivate werden unter der Handelsbezeichnung Jaguar^{®} vertrieben und haben die INCI-Bezeichnung Guar Hydroxypropyltrimonium Chloride. Weiterhin sind besonders geeignete kationische Guarderivate auch von der Fa. Hercules unter der Bezeichnung N-Hance^{®} im Handel. Weitere kationische Guarderivate werden von der Fa. Cognis unter der Bezeichnung Cosmedia^{®} vertrieben. Ein bevorzugtes kationisches Guarderivat ist das Handelsprodukt AquaCat^{®} der Fa. Hercules. Bei diesem Rohstoff handelt es sich um ein bereits vorgelöstes kationisches Guarderivat. Die kationischen Guar-Derivate sind erfindungsgemäß bevorzugt.

Ein geeignetes Chitosan wird beispielsweise von der Firma Kyowa Oil& Fat, Japan, unter dem Handelsnamen Flonac^{®} vertrieben. Ein bevorzugtes Chitosansalz ist Chitosoniumpyrrolidoncarboxylat, welches beispielsweise unter der Bezeichnung Kytamer^{®} PC von der Firma Amerchol, USA, vertrieben wird. Weitere Chitosanderivate sind unter den Handelsbezeichnungen Hydagen^{®} CMF, Hydagen^{®} HCMF und Chitolam^{®} NB/101 im Handel frei verfügbar. Schließlich sind auch kationische Polymere auf der Basis von Zuckern erfindungsgemäß mit Vorzug verwendbar.

Derartige Verbindungen sind beispielsweise kationische Alkyloligoglucoside wie in der folgenden Abbildung gezeigt.

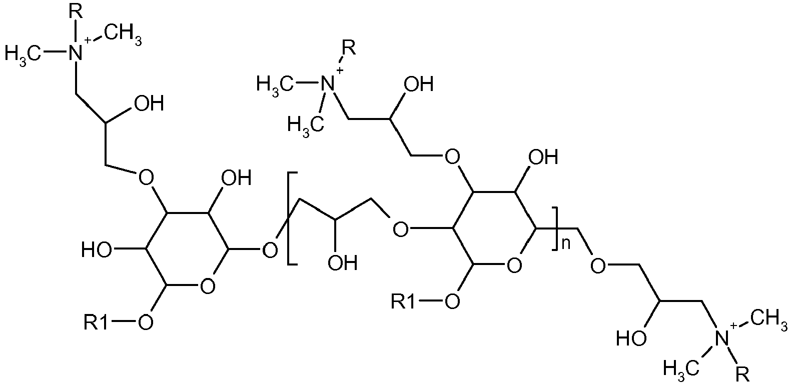

In der zuvor dargestellten Formel stehen die Reste R unabhängig voneinander für einen linearen oder verzweigten C6 bis C30 Alkylrest, einen linearen oder verzweigten C6 - C30 Alkenylrest, vorzugsweise steht der Rest R für einen Rest R ausgewählt aus: Lauryl, Myristyl, Cetyl, Stearyl, Oleyl, Behenyl oder Arachidyl.

Die Reste R1 stehen unabhängig voneinander für einen linearen oder verzweigten C6 bis C30 Alkylrest, einen linearen oder verzweigten C6 bis C30 Alkenylrest, vorzugsweise steht der Rest R für einen Rest ausgewählt aus: Butyl, Capryl, Caprylyl, Octyl, Nonyl, Decanyl, Lauryl, Myristyl, Cetyl, Stearyl, Oleyl, Behenyl oder Arachidyl. Besonders bevorzugt sind die Reste R1 gleich. Noch bevorzugter sind die Reste R1 ausgewählt aus technischen Mischungen der Fettalkoholschnitte aus C6/C8 - Fettalkoholen, C8/C10 - Fettalkoholen, C10/C12 - Fettalkoholen, C12/C14 - Fettalkoholen, C12 / C18 - Fettalkoholen, und höchst bevorzugt sind hierbei diejenigen technischen Fettalkoholschnitte, welche pflanzlichen Ursprunges sind.

Besonders bevorzugte Beispiele für die kationischen Alkyloligoglucoside sind die Verbindungen mit den INCI - Bezeichnungen Polyquaternium-77, Polyquaternium-78, Polyquaternium-79, Polyquaternium-80, Polyquaternium-81 und Polyquaternium-82. Höchst bevorzugt sind die kationischen Alkyloligoglucoside mit den Bezeichnungen Polyquaternium-77, Polyquaternium-81 und Polyquaternium-82.

Derartige Verbindungen können beispielsweise unter der Bezeichnung Poly Suga® Quat von der Fa. Colonial Chemical Inc. bezogen werden.

Die kationischen Alkyloligoglucoside werden in einer Gesamtmenge von 0,01 bis 10,0 Gew.%, vorzugsweise von 0,05 bis 5,0 Gew.%, noch bevorzugter von 0,1 bis 3,0 Gew.% und höchst bevorzugt in Mengen von 0,2 bis 2,0 Gew.% jeweils bezogen auf das Gesamtgewicht der Zusammensetzung verwendet. Erfindungsgemäß umfaßt ist selbstverständlich auch, dass Mischungen von kationischen Alkyloligoglucosiden verwendet werden können. Bevorzugt ist in diesem Falle, wenn jeweils ein langkettiges und ein kurzkettiges kationisches Alkyloligoglucosid gleichzeitig verwendet werden.

Ein weiteres kationisches Polymer kann auf der Grundlage von Ethanolamin erhalten werden. Das Polymer ist unter der Bezeichnung Polyquaternium-71 im Handel erhältlich.

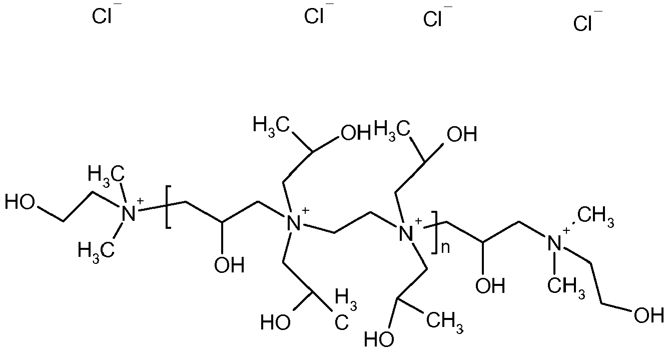

Dieses Polymer kann beispielsweise unter der Bezeichnung Cola® Moist 300 P von der Fa. Colonial Chemical Inc. bezogen werden.

Das Polyquaternium-71 wird in einer Gesamtmenge von 0,01 bis 10,0 Gew.%, vorzugsweise von 0,05 bis 5,0 Gew.%, noch bevorzugter von 0,1 bis 3,0 Gew.% und höchst bevorzugt in Mengen von 0,2 bis 2,0 Gew.% jeweils bezogen auf das Gesamtgewicht der Zusammensetzung verwendet. Weitere bevorzugte kationische Polymere sind beispielsweise
- kationisierter Honig, beispielsweise das Handelsprodukt Honeyquat^{®} 50,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere mit der INCI - Bezeichnung Polyquaternium-7,
- Vinylpyrrolidon-Vinylimidazoliummethochlorid-Copolymere, wie sie unter den Bezeichnungen Luviquat^{®} FC 370, FC 550 und der INCI-Bezeichnung Polyquaternium-16 sowie FC 905 und HM 552 angeboten werden,
- quaternisiertes Vinylpyrrolidon/Dimethylaminoethylmethacrylat, zum Beispiel Vinylpyrrolidon/Dimethylaminoethylmethacrylatmethosulfat Copolymer, das unter den Handelsbezeichnungen Gafquat^{®} 755 N und Gafquat^{®} 734 von der Firma Gaf Co., USA vertrieben wird und die INCI - Bezeichnung Polyquaternium-11,
- quaternierter Polyvinylalkohol,
- sowie die unter den Bezeichnungen Polyquaternium-2, Polyquaternium-17, Polyquaternium-18 und Polyquaternium-27 bekannten Polymeren mit quartären Stickstoffatomen in der Polymerhauptkette,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Erfindungsgemäße amphotere Polymere sind solche Polymerisate, in denen sich eine kationische Gruppe ableitet von mindestens einem der folgenden Monomere:
(i) Monomeren mit quartären Ammoniumgruppen der allgemeinen Formel (Mono1),

   R¹-CH=CR²-CO-Z-(CₙH₂ₙ)-N⁽⁺⁾R²R³R⁴ A⁽⁻⁾ (Mono1)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist,
(ii) Monomeren mit quartären Ammoniumgruppen der allgemeinen Formel (Mono2), 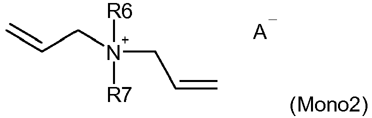
   worin R⁶ und R⁷ unabhängig voneinander stehen für eine (C₁ bis C₄)-Alkylgruppe, insbesondere für eine Methylgruppe und
   A⁻ das Anion einer organischen oder anorganischen Säure ist,
(iii) monomeren Carbonsäuren der allgemeinen Formel (Mono3),

   R⁸-CH=CR⁹-COOH (Mono3)

   in denen R⁸ und R⁹ unabhängig voneinander Wasserstoff oder Methylgruppen sind.
   Besonders bevorzugt sind solche Polymerisate, bei denen Monomere des Typs (i) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (i). Als Monomeres (ii) für die genannten Polymerisate wird bevorzugt Acrylsäure verwendet.
   Besonders bevorzugte amphotere Polymere sind Copolymere, aus mindestens einem Monomer (Mono1) bzw. (Mono2) mit dem Momomer (Mono3), insbesondere Copolymere aus den Monomeren (Mono2) und (Mono3). Erfindungsgemäß ganz besonders bevorzugt verwendete amphotere Polymere sind Copolymerisate aus Diallyl-dimethylammoniumchlorid und Acrylsäure. Diese Copolymerisate werden unter der INCI-Bezeichnung Polyquaternium-22 unter anderem mit dem Handelsnamen Merquat^{®} 280 (Nalco) vertrieben.
   Darüber hinaus können die erfindungsgemäßen amphoteren Polymere neben einem Monomer (Mono1) oder (Mono2) und einem Monomer (Mono3) zusätzlich ein Monomer (Mono4)
(iv) monomere Carbonsäureamide der allgemeinen Formel (Mono4), 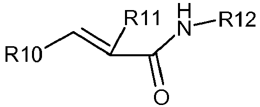 in denen R¹⁰ und R¹¹ unabhängig voneinander Wasserstoff oder Methylgruppen sind und R¹² für ein Wasserstoffatom oder eine (C₁- bis C₈)-Alkylgruppe steht, enthalten.

Erfindungsgemäß ganz besonders bevorzugt verwendete amphotere Polymere auf Basis eines Comonomers (Mono4) sind Terpolymere aus Diallyldimethylammoniumchlorid, Acrylamid und Acrylsäure. Diese Copolymerisate werden unter der INCI-Bezeichnung Polyquaternium-39 unter anderem mit dem Handelsnamen Merquat^{®} Plus 3330 (Nalco) vertrieben.

Die amphoteren Polymere können generell sowohl direkt als auch in Salzform, die durch Neutralisation der Polymerisate, beispielsweise mit einem Alkalihydroxid, erhalten wird, erfindungsgemäß eingesetzt werden.

Die zuvor genannten kationischen Polymere können einzeln oder in beliebigen Kombinationen miteinander verwendet werden, wobei Mengen zwischen 0,01 bis 10 Gew.%, bevorzugt, Mengen von 0,01 bis 7,5 Gew.% und ganz besonders bevorzugt in Mengen von 0,1 bis 5,0 Gew.% enthalten sind. Die allerbesten Ergebnisse werden dabei mit Mengen von 0,1 bis 3,0 Gew.% jeweils bezogen auf die Gesamtzusammensetzung des jeweiligen Mittels erhalten.

Weiterhin enthalten die erfindungsgemäßen Mittel bevorzugt mindestens ein Silikonpolymer ausgewählt aus der Gruppe der Dimethiconole und/oder der Gruppe der aminofunktionellen Silikone und/oder der Gruppe der Dimethicone und / oder der Gruppe der Cyclomethicone. Für jede dieser Gruppen gilt jedoch, dass arylierte Silikone der jeweiligen Gruppen von Silikonen, nicht in den erfindungsgemäßen Zusammensetzungen enthalten sind.

Die erfindungsgemäßen Dimethicone können sowohl linear als auch verzweigt als auch cyclisch oder cyclisch und verzweigt sein. Lineare Dimethicone können durch die folgende Strukturformel (Si1) dargestellt werden:

(SiR¹₃) - O - (SiR²₂ - O -)ₓ - (SiR¹₃) (Si1)

Verzweigte Dimethicone können durch die Strukturformel (Si1.1) dargestellt werden:

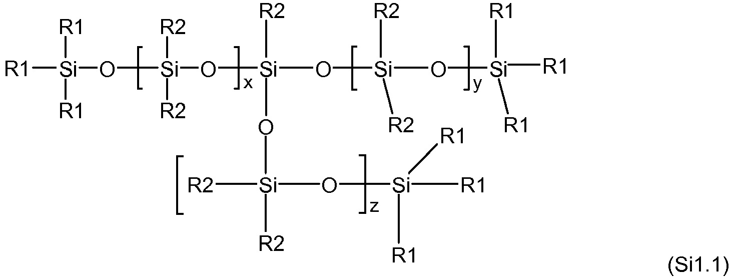

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs. Höchst bevorzugt sind Viskositäten um den Bereich von etwa 60.000 cPs herum. Beispielhaft sei hier auf das Produkt "Dow Corning 200 mit 60000cSt" verwiesen.

Besonders bevorzugte erfindungsgemäße kosmetische oder dermatologische Zubereitungen sind dadurch gekennzeichnet, dass sie mindestens ein Silikon der Formel (Si1.2)

(CH₃)₃Si-[O-Si(CH₃)₂]ₓ-O-Si(CH₃)₃ (Si1.2),

enthalten, in der x für eine Zahl von 0 bis 100, vorzugsweise von 0 bis 50, weiter bevorzugt von 0 bis 20 und insbesondere 0 bis 10, steht.

Die Dimethicone (Si1) sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% bezogen auf die gesamte Zusammensetzung enthalten. Schließlich werden unter den Silikonverbindungen die Dimethiconole (Si8) verstanden. Die erfindungsgemäßen Dimethiconole können sowohl linear als auch verzweigt als auch cyclisch oder cyclisch und verzweigt sein. Lineare Dimethiconole können durch die folgende Strukturformel (Si8 - I) dargestellt werden:

(SiOHR¹₂) - O - (SiR²₂ - O -)ₓ - (SiOHR¹₂) (Si8 - I)

Verzweigte Dimethiconole können durch die Strukturformel (Si8 - II) dargestellt werden:

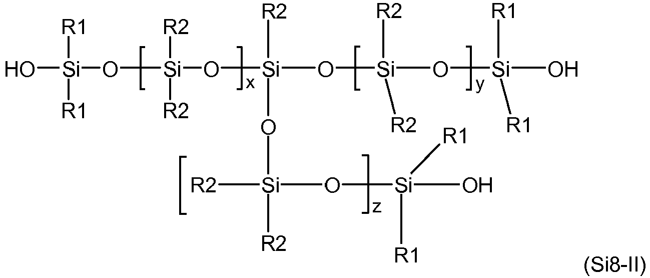

Die Reste R¹ und R² stehen unabhängig voneinander jeweils für Wasserstoff, einen Methylrest, einen C2 bis C30 linearen, gesättigten oder ungesättigten Kohlenwasserstoffrest. Die Zahlen x, y und z sind ganze Zahlen und laufen jeweils unabhängig voneinander von 0 bis 50.000. Die Molgewichte der Dimethiconole liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Als Beispiele für derartige Produkte werden die folgenden Handelsprodukte genannt: Dow Corning 1-1254 Fluid, Dow Corning 2-9023 Fluid, Dow Corning 2-9026 Fluid, Abil OSW 5 (Degussa Care Specialties), Dow Corning 1401 Fluid, Dow Corning 1403 Fluid, Dow Corning 1501 Fluid, Dow Corning 1784 HVF Emulsion, Dow Corning 9546 Silicone Elastomer Blend, SM555, SM2725, SM2765, SM2785 (alle vier zuvor genannten GE Silicones), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH). Die Dimethiconole (Si8) sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Dimethiconol bezogen auf die Zusammensetzung.

Besonders bevorzugte erfindungsgemäße Mittel enthalten ein oder mehrere aminofunktionelle Silikone. Solche Silikone können z.B. durch die Formel (Si-2)

M(RₐQ_{b}SiO_{(4-a-b)/2})ₓ(R_{c}SiO_{(4-c)/2})_{y}M (Si-2)

Beschrieben werden, wobei in der obigen Formel
- R: ein Kohlenwasserstoff oder ein Kohlenwasserstoffrest mit 1 bis etwa 6 Kohlenstoffatomen ist,
- Q: ein polarer Rest der allgemeinen Formel -R¹HZ ist, worin
R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und
Z ein organischer, aminofunktioneller Rest ist, der mindestens eine aminofunktionelle Gruppe enthält;
- a: Werte im Bereich von etwa 0 bis etwa 2 annimmt,
- b: Werte im Bereich von etwa 1 bis etwa 3 annimmt,
- a + b: kleiner als oder gleich 3 ist, und
- c: eine Zahl im Bereich von etwa 1 bis etwa 3 ist, und
- x: eine Zahl im Bereich von 1 bis etwa 2.000, vorzugsweise von etwa 3 bis etwa 50 und am bevorzugtesten von etwa 3 bis etwa 25 ist, und
- y: eine Zahl im Bereich von etwa 20 bis etwa 10.000, vorzugsweise von etwa 125 bis etwa 10.000 und am bevorzugtesten von etwa 150 bis etwa 1.000 ist, und
- M: eine geeignete Silikon-Endgruppe ist, wie sie im Stande der Technik bekannt ist, vorzugsweise Trimethylsiloxy.

Z ist gemäß Formel (Si-2) ein organischer, aminofunktioneller Rest, enthaltend mindestens eine funktionelle Aminogruppe. Eine mögliche Formel für besagtes Z ist NH(CH₂)_{z}NH₂, worin z eine ganze Zahl von größer gleich 1 ist. Eine andere mögliche Formel für besagtes Z ist -NH(CH₂)_{z}(CH ₂)_{zz}NH, worin sowohl z als auch zz unabhängig voneinander eine ganze Zahl von größer gleich 1 sind, wobei diese Struktur Diamino-Ringstrukturen umfasst, wie Piperazinyl. Besagtes Z ist am bevorzugtesten ein -NHCH₂CH ₂NH₂-Rest. Eine andere mögliche Formel für besagtes Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q gemäß Formel (Si-2) ist am bevorzugtesten ein polarer aminofunktioneller Rest der Formel - CH₂CH₂CH₂NHCH₂CH₂NH₂.

In der Formel (Si-2) nimmt a Werte im Bereich von 0 bis 2 an, b nimmt Werte im Bereich von 2 bis 3 an, a + b ist kleiner als oder gleich 3, und c ist eine Zahl im Bereich von 1 bis 3. Erfindungsgemäß geeignet sind kationische Silikonöle wie beispielsweise die im Handel erhältlichen Produkte Dow Corning (DC) 929 Emulsion, DC 2-2078, DC 5-7113, SM-2059 (General Electric) sowie SLM-55067 (Wacker).

Besonders bevorzugte erfindungsgemäße Mittel sind dadurch gekennzeichnet, dass sie mindestens es ein aminofunktionelles Silikon der Formel (Si3-a) 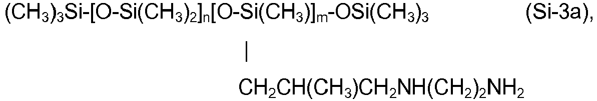 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet und sind beispielsweise unter der Bezeichnung Q2-7224 (Hersteller: Dow Corning; ein stabilisiertes Trimethylsilylamodimethicon) erhältlich.

Besonders bevorzugt sind auch erfindungsgemäße Mittel, die mindestens ein aminofunktionelles Silikon der Formel (Si-3b) 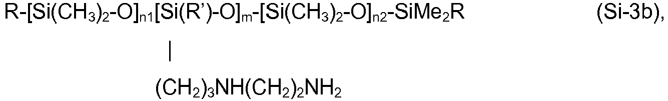 enthalten, worin
- R: für -OH, eine (gegebenenfalls ethoxylierte und/oder propoxylierte) (C₁ bis C₂₀)-Alkoxygruppe oder eine -CH₃-Gruppe steht,
- R': für -OH, eine (C₁ bis C₂₀)-Alkoxygruppe oder eine -CH₃-Gruppe und
- m, n1 und n2: Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Amodimethicone, bzw. als funktionalisierte Amodimethicone, wie beispielsweise Bis(C13-15 Alkoxy) PG Amodimethicone (beispielsweise als Handelsprodukt: DC 8500 der Firma Dow Corning erhältlich), Trideceth-9 PG-Amodimethicone (beispielsweise als Handelsprodukt Silcare Silicone SEA der Firma Clariant erhältlich) bezeichnet. Geeignete diquaternäre Silikone sind ausgewählt aus Verbindungen der allgemeinen Formel (Si3c)

[R¹R ²R³N⁺ - A - SiR⁷R⁸- (O-SiR⁹R¹⁰)ₙ- O - SiR¹¹R¹² - A - N⁺R⁴R⁵R⁶] 2X⁻ (Si3c)

wobei die Reste R1 bis R6 unabhängig voneinander C1-bis C22-Alkylreste bedeuten, welche Hydroxygruppen enthalten können und wobei vorzugsweise mindestens einer der Reste mindestens 8 C-Atome aufweist und die übrigen Reste 1 bis 4 C-Atome aufweisen,
die Reste R7 bis R12 unabhängig voneinander gleich oder verschieden sind und C1-bis C10-Alkyl bedeuten, A eine divalente organische Verbindungsgruppe bedeutet,
n eine Zahl von 0 bis 200, vorzugsweise von 10 bis 120, besonders bevorzugt von 10 bis 40 ist, und X⁻ ein Anion ist.

Die divalente Verbindungsgruppe ist vorzugsweise eine C1-bis C12-Alkylen-oder Alkoxyalkylengruppe, die mit einer oder mehreren Hydroxylgruppen substituiert sein kann. Besonders bevorzugt ist die Gruppe -(CH₂)₃-O-CH₂-CH(OH)-CH₂-.

Das Anion X⁻ kann ein Halogenidion, ein Acetat, ein organisches Carboxylat oder eine Verbindung der allgemeinen Formel RSO₃⁻ sein, worin R die Bedeutung von C1-bis C4-Alkylresten hat.

Ein bevorzugtes diquaternäres Silikon hat die allgemeine Formel (Si3d)

[RN⁺Me₂ - A - (SiMe₂O)ₙ-SiMe₂ - A - N⁺Me₂R] 2 CH₃COO⁻ (Si3d),

wobei A die Gruppe -(CH₂)₃- O - CH₂ - CH(OH) - CH₂ -ist,
R ein Alkylrest mit mindestens 8 C-Atomen und n eine Zahl von 10 bis 120 ist.

Geeignete Silikonpolymere mit zwei endständigen, quaternären Ammoniumgruppen sind unter der INCI-Bezeichnung Quaternium-80 bekannt. Hierbei handelt es sich um Dimethylsiloxane mit zwei endständigen Trialkylammoniumgruppen. Derartige diquaternäre Polydimethylsiloxane werden von der Firma Evonik unter den Handelsnamen Abil^{®} Quat 3270, 3272 und 3474 vertrieben. Erfindungsgemäß bevorzugte Haarbehandlungsmittel sind dadurch gekennzeichnet, dass sie, bezogen auf ihr Gewicht, 0,01 bis 10 Gew.%, vorzugsweise 0,01 bis 8 Gew.%, besonders bevorzugt 0,1 bis 7,5 Gew.% und insbesondere 0,2 bis 5 Gew.% aminofunktionelle(s) Silikon(e) und/oder diquaternäres Silikon enthalten.

Ein weiteres erfindungsgemäßes Silikon mit Aminofunktionen sind Polyammonium-Polysiloxan Verbindungen. Die Polyammonium-Polysiloxan Verbindungen können beispielsweise unter der Handelsbezeichnung Baysilone® von GE Bayer Silicones bezogen werden. Die Produkte mit den Bezeichnungen Baysilone TP 3911, SME 253 und SFE 839 sind dabei bevorzugt. Ganz besonders bevorzugt ist die Verwendung von Baysilone TP 3911 als Wirkkomponente der erfindungsgemäßen Zusammensetzungen. Die Polyammonium-Polysiloxan Verbindungen werden in dem erfindungsgemäßen Zusammensetzungen in einer Menge von 0,01 bis 10 Gew.% , vorzugsweise 0,01 bis 7,5, besonders bevorzugt 0,01 bis 5,0 Gew.%, ganz besonders bevorzugt von 0,05 bis 2,5 Gew.% jeweils in Bezug auf die Gesamtzusammensetzung verwendet.

Die EP 1887024 A1 beschreibt neuartige kationische aminofunktionelle Silikone, welche insbesondere den Glanz in Mitteln zur Pflege von Oberflächen, beispielsweise menschlichen Haaren, verbessern. Diese kationischen Silikonpolymere zeichnen sich dadurch aus, dass sie ein Silikongerüst sowie mindestens einen Polyetherteil und weiterhin mindestens einen Teil mit Ammoniumstruktur aufweisen. Beispiele für die bevorzugten kationischen Silikonpolymere im Sinne der vorliegenden Erfindung sind neben den Verbindungen der zuvor genannten EP 1887024 A1 weiterhin insbesondere die Verbindungen mit den INCI - Bezeichnungen: Silicone Quaternium-1, Silicone Quaternium-2, Silicone Quaternium-3, Silicone Quaternium-4, Silicone Quaternium-5, Silicone Quaternium-6, Silicone Quaternium-7, Silicone Quaternium-8, Silicone Quaternium-9, Silicone Quaternium-10, Silicone Quaternium-11, Silicone Quaternium-12, Silicone Quaternium-15, Silicone Quaternium-16, Silicone Quaternium-17, Silicone Quaternium-18, Silicone Quaternium-20, Silicone Quaternium-21, Silicone Quaternium-22 sowie Silicone Quaternium-2 Panthenol Succinate und Silicone Quaternium-16/Glycidyl Dimethicone Crosspolymer. Am bevorzugtesten ist insbesondere Silicone Quaternium-22. Dieses Rohstoff wird beispielsweise von der Firma Evonik unter der Handelsbezeichnung Abil^{®} T-Quat 60 vertrieben.

Die kationischen aminofunktionellen Silikonpolymere sind in den erfindungsgemäßen Zusammensetzungen in Mengen von 0,01 bis 20 Gew.%, bevorzugt in Mengen von 0,05 bis 10 Gew.% und ganz besonders bevorzugt in Mengen von 0,1 bis 7,5 Gew.% enthalten. Die allerbesten Ergebnisse werden dabei mit Mengen von 0,1 bis 5 Gew.% jeweils bezogen auf die Gesamtzusammensetzung des jeweiligen Mittels erhalten.

Auch die nach INCI als Cyclomethicone bezeichneten cyclischen Dimethicone sind erfindungsgemäß mit Vorzug einsetzbar. Hier sind erfindungsgemäße kosmetische oder dermatologische Zubereitungen bevorzugt, die mindestens ein Silikon der Formel (Si-4) 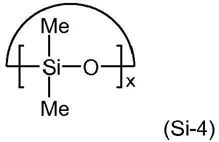 enthalten, in der x für eine Zahl von 3 bis 200, vorzugsweise von 3 bis 10, weiter bevorzugt von 3 bis 7 und insbesondere 3, 4, 5 oder 6, steht.

Erfindungsgemäß ebenfalls bevorzugte Mittel sind dadurch gekennzeichnet, dass sie mindestens ein Silikon der Formel (Si-5)

R₃Si-[O-SiR₂]ₓ-(CH₂)ₙ-[O-SiR₂]_{y}-O-SiR₃ (Si-5),

enthalten, in der R für gleiche oder verschiedene Reste aus der Gruppe -H, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, - CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Als weitere Silikone neben den erfindungsgemäßen Dimethiconen, Dimethiconolen, Amodimethiconen und/oder Cyclomethiconen können wasserlösliche Silikone in den erfindungsgemäßen Zusammensetzungen enthalten sein.

Entsprechende hydrophile Silikone werden beispielsweise aus den Verbindungen der Formeln (Si-6) und/oder (Si-7) ausgewählt. Insbesondere bevorzugte wasserlösliche Tenside auf Silikonbasis sind ausgewählt aus der Gruppe der Dimethiconcopolyole die bevorzugt alkoxyliert, insbesondere polyethoxyliert oder polypropoxyliert sind.

Unter Dimethiconcopolyolen werden erfindungsgemäß bevorzugt Polyoxyalkylen-modifizierte Dimethylpolysiloxane der allgemeinen Formeln (Si-6) oder (Si-7) verstanden: 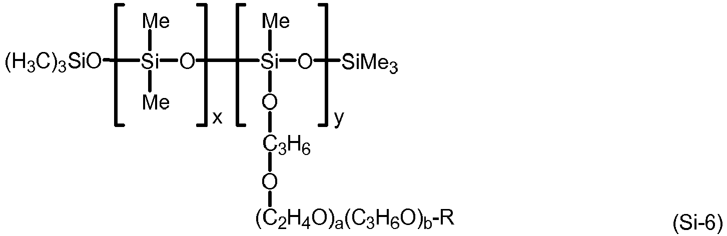 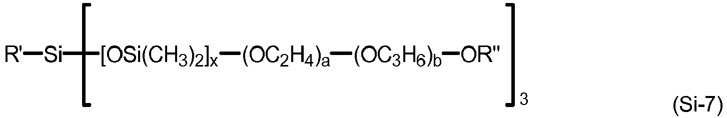 worin der Rest R steht für ein Wasserstoffatom, eine Alkylgruppe mit 1 bis 12 C-Atomen, eine Alkoxygruppe mit 1 bis 12 C-Atomen oder eine Hydroxylgruppe, die Reste R' und R" bedeuten Alkylgruppen mit 1 bis 12 C-Atomen, x steht für eine ganze Zahl von 1 bis 100, bevorzugt von 20 bis 30, y steht für eine ganze Zahl von 1 bis 20, bevorzugt von 2 bis 10 und a und b stehen für ganze Zahlen von 0 bis 50, bevorzugt von 10 bis 30.

Besonders bevorzugte Dimethiconcopolyole im Sinne der Erfindung sind beispielsweise die kommerziell unter dem Handelsnamen SILWET (Union Carbide Corporation) und DOW CORNING vertriebenen Produkte. Erfindungsgemäß besonders bevorzugte Dimethiconcopolyole sind Dow Corning 190 und Dow Corning 193.

Die Dimethiconcopolyole sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Dimethiconcopolyol bezogen auf die Zusammensetzung.

Ein weiterer pflegender Inhaltsstoff, welcher mit besonderem Vorzug verwendet werden kann, ist ein Esteröl. Esterölen wird in den bevorzugten erfindungsgemäßen Zusammensetzungen der Vorzug vor den Silikonölen gewährt. Die Esteröle sind wie folgt definiert:
Unter Esterölen sind zu verstehen die Ester von C₆ - C₃₀ - Fettsäuren mit C₂ - C₃₀ - Fettalkoholen.

Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®}868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).

Selbstverständlich können die Esteröle auch mit Etyhlenoxid, Propylenoxid oder Mischungen aus Ethylenoxid und Propylenoxid alkoxiliert sein. Die Alkoxylierung kann dabei sowohl am Fettalkoholpart als auch am Fettsäurepart als auch an beiden Teilen der Esteröle zu finden sein.

Bevorzugt ist erfindungsgemäß jedoch, wenn zunächst der Fettalkohol alkoxyliert wurde und anschließend mit Fettsäure verestert wurde. In der Formel (D4-II) sind allgemein diese Verbindungen dargestellt. 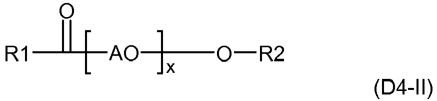
R1 steht hierbei für einen gesättigten oder ungesättigten, verzweigten oder unverzweigten, cyclischen gesättigten cyclischen ungesättigten Acylrest mit 6 bis 30 Kohlenstoffatomen,
AO steht für Ethylenoxid, Propylenoxid oder Butylenoxid,
X steht für eine Zahl zwischen 1 und 200, vorzugsweise 1 und 100, besonders bevorzugt zwischen 1 und 50, ganz besonders bevorzugt zwischen 1 und 20, höchst bevorzugt zwischen 1 und 10 und am bevorzugtesten zwischen 1 und 5,
R2 steht für einen gesättigten oder ungesättigten, verzweigten oder unverzweigten, cyclischen gesättigten cyclischen ungesättigten Alkyl-, Alkenyl-, Alkinyl-, Phenyl- oder Benzylrest mit 6 bis 30 Kohlenstoffatomen. Beispiele für eingesetzte Fettsäurenanteile als Rest R1 in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile als Rest R2 in den Esterölen sind Benzylalkohol, Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Ein erfindungsgemäß besonders bevorzugtes Esteröl ist beispielsweise unter der INCI - Bezeichnung PPG-3 Benzyl Ether Myristate erhältlich.

Weiterhin sind unter Esterölen zu verstehen:
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat, sowie
- symmetrische, unsymmetrische oder cyclische Ester der Kohlensäure mit Fettalkoholen, beispielsweise Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, das sind Monoglyceride, Diglyceride und deren technische Gemische. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Esteröle werden in den erfindungsgemäßen Mitteln in einer Menge von 0,01 bis 20 Gew.%, bevorzugt 0,01 bis 10,0 Gew.%, besonders bevorzugt 0,01 bis 7,5 Gew.%, höchst bevorzugt von 0,1 bis 5,0 Gew.% verwendet. Selbstverständlich ist es erfindungsgemäß auch möglich mehrere Esteröle gleichzeitig zu verwenden.

Weiterer erfindungsgemäße Ölkörper sind:
- pflanzliche Öle. Beispiele für solche Öle sind Arganöl, Aprikosenkernöl, Marulaöl, Macadamiaöl, Sesamöl, Sonnenblumenöl, Olivenöl, Sojaöl, Rapsöl, Mandelöl, Jojobaöl, Orangenöl, Kapkastanienöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle wie Coco caprylate,
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.

Als natürliche Öle werden beispielsweise Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Haselnussöl, Holundersamenöl, Johannesbeersamenöl, Jojobaöl, Kakaobutter, Leinöl, Macadamianussöl, Maiskeimöl, Mandelöl, Marulaöl, Nachtkerzenöl, Olivenöl, Palmöl, Rapsöl, Reisöl, Sanddornfruchtfleischöl, Sanddornkernöl, Sesamöl, Sheabutter, Sojaöl, Sonnenblumenöl, Traubenkernöl, Walnußöl oder Wildrosenöl verwendet.

Besonders bevorzugt sind aus den vorgenannten Esterölen Amaranthsamenöl, Aprikosenkernöl, Arganöl, Marulaöl, Macadamiaöl, Mandelöl, insbesondere Süßmandelöl, Sesamöl, Sonnenblumenöl, Olivenöl, Orangenöl, Kapkastanienöl, Weizenkeimöl, Pfirsichkernöl, Isopropylmyristate, Dicaprylylcarbonate und/oder Coco-caprylate sowie deren Mischungen.

Diese besonders bevorzugten Esteröle werden in den erfindungsgemäßen Mitteln in einer Menge von 0,01 bis 20 Gew.%, bevorzugt 0,01 bis 10,0 Gew.%, besonders bevorzugt 0,01 bis 7,5 Gew.%, höchst bevorzugt von 0,1 bis 5,0 Gew.% verwendet. Selbstverständlich ist es erfindungsgemäß auch möglich mehrere Esteröle gleichzeitig zu verwenden.

Selbstverständlich enthalten die erfindungsgemäßen Haarbehandlungsmittel neben der erfindungsgemäßen Wirkstoffkombination auch weitere in kosmetischen Zusammensetzungen übliche Bestandteile. Die Auswahl dieser Bestandteile richtet sich im allgemeinen nach der beabsichtigten Verwendung der Haarbehandlungsmittel. Im Falle eines Shampoos werden beispielsweise weitere oberflächenaktive Substanzen enthalten sein. Im Falle von Haarkuren werden gegebenenfalls weitere kationische Verbindungen und weitere Pflegestoffe enthalten sein. In vielen Fällen enthalten die Mittel mindestens eine oberflächenaktive Substanz, wobei prinzipiell sowohl anionische als auch zwitterionische, ampholytische, nichtionische und kationische oberflächenaktive Substanzen geeignet sind. Die Auswahl der oberflächenaktiven Substanzen richtet sich nach der Art des Mittels.

Als anionische Tenside (Tanion) eignen sich in erfindungsgemäßen Zubereitungen alle für die Verwendung am menschlichen Körper geeigneten anionischen oberflächenaktiven Stoffe. Typische Beispiele für anionische Tenside sind:
- lineare und verzweigte Fettsäuren mit 8 bis 30 C-Atomen (Seifen),
- Ethercarbonsäuren der Formel R-O-(CH₂CH₂O)ₓ-CH₂-COOH, in der R eine lineare Alkylgruppe mit 8 bis 30 C-Atomen und x = 0 oder 1 bis 16 ist und deren Salze,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen.
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Hydroxysulfonate im wesentlichen entsprechend mindestens einer der beiden folgenden Formeln oder deren deren Mischungen sowie deren salzen, CH₃-(CH₂)_{y}-CHOH-(CH₂)ₚ-(CH-SO₃M)-(CH₂)_{z}-CH₂-O-(CₙH₂ₙO)ₓ-H, und/oder CH₃-(CH₂)_{y}-(CH-SO₃M)-(CH₂)ₚ-CHOH-(CH₂)_{z}- CH₂-O-(CₙH₂ₙO)ₓ-H wobei in beiden Formeln y und z = 0 oder ganze Zahlen von 1 bis 18, p = 0, 1 oder 2 und die Summe (y+z+p) eine Zahl von 12 bis 18, x = 0 oder eine Zahl von 1 bis 30 und n eine ganze Zahl von 2 bis 4 sowie M = H oder Alkali-, insbesondere Natrium, Kalium, Lithium, Erdalkali- , insbesondere Magnesium, Calcium, Zink und/oder einem Ammoniumion, welches gegebenenfalls substituiert sein kann, insbesondere Mono-, Di-, Tri- oder Tetraammoniumionen mit C1 bis C4 Alkyl-, Alkenyl- oder Arylresten,
- sulfatierte Hydroxyalkylpolyethylen- und/oder Hydroxyalkylenpropylenglykolether der Formel R¹-(CHOSO₃M)-CHR³-(OCHR⁴-CH₂)n-OR² mit R¹, einem linearen Alkylrest mit 1 bis 24 C-Atomen, R² für einen linearen oder verzweigten, gesättigten Alkylrest mit 1 bis 24 C-Atomen, R³ für Wasserstoff oder einen linearen Alkylrest mit 1 bis 24 C-Atomen, R⁴ für Wasserstoff oder einen Methylrest und M für Wasserstoff, Ammonium, Alkylammonium, Alkanolammonium, worin die Alkyl- und Alkanolreste je 1 bis 4 C-Atome aufweisen, oder ein Metallatom ausgewählt aus Lithium, Natrium, Kalium, Calcium oder Magnesium und n für eine Zahl im Bereich von 0 bis 12 stehen und weiterhin die Gesamtzahl der in R¹ und R³ enthaltenen C-Atome 2 bis 44 beträgt,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel,

   R¹(OCH₂CH₂)ₙ-O-(PO-OX)-OR²,

   in der R¹ bevorzugt für einen aliphatischen Kohlenwasserstoffrest mit 8 bis 30 Kohlenstoffatomen, R² für Wasserstoff, einen Rest (CH₂CH₂O)ₙR² oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄ - Kohlenwasserstoffrest, steht,
- sulfatierte Fettsäurealkylenglykolester der Formel RCO(AlkO)ₙSO₃M in der RCO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Metall steht, wie Alkalimetall, insbesondere Natrium, Kalium, Lithium, Erdalkalimetall, insbesondere Magnesium, Calcium, Zink, oder Ammoniumion, wie ⁺NR³R⁴R⁵R⁶, mit R³ bis R⁶ unabhängig voneinander stehend für Wasserstoff oder einen C1 bis C4 - Kohlenwasserstoffrest,
- Monoglyceridsulfate und Monoglyceridethersulfate der Formel

   R⁸OC-(OCH₂CH₂)ₓ-OCH₂-[CHO(CH₂CH₂O)_{y}H]-CH₂O(CH₂CH₂O)_{z}-SO₃X,

   in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate eingesetzt, in der R⁸CO für einen linearen Acylrest mit 8 bis 18 Kohlenstoffatomen steht,
- Amidethercarbonsäuren, R¹-CO-NR²-CH₂CH₂-O-(CH₂CH₂O)ₙCH₂COOM, mit R¹ als geradkettigen oder verzweigten Alkyl- oder Alkenylrest mit einer Zahl an Kohlenstoffatomen in der Kette von 2 bis 30, n steht für eine ganze Zahl von 1 bis 20 und R² steht für Wasserstoff, einen Methyl-, Ethyl-, Propyl-, Isopropyl-, n-Butyl-, t-Butyl- oder iso-Butylrest und M steht für Wasserstoff oder ein Metall wie Alkalimetall, insbesondere Natrium, Kalium, Lithium, Erdalkalimetall, insbesondere Magnesium, Calcium, Zink, oder ein Ammoniumion, wie ⁺NR³R⁴R⁵R⁶, mit R³ bis R⁶ unabhängig voneinander stehend für Wasserstoff oder einen C1 bis C4 - Kohlenwasserstoffrest. Derartige Produkte sind beispielsweise von der Firma Chem-Y unter der Produktbezeichnung Akypo^{®} erhältlich.
- Acylglutamate der Formel XOOC-CH2CH2CH(C(NH)OR)-COOX, in der RCO für einen linearen oder verzweigten Acylrest mit 6 bis 22 Kohlenstoffatomen und 0 und/oder 1, 2 oder 3 Doppelbindungen und X für Wasserstoff, ein Alkali- und/oder Erdalkalimetall, Ammonium, Alkylammonium, Alkanolammonium oder Glucammonium steht,
- Kondensationsprodukte aus einem wasserlöslichen Salz eines wasserlöslichen Eiweißhydrolysats mit einer C8 - C30 - Fettsäure. Solche Produkte sind unter dem Warenzeichen Lamepon^{®}, Maypon^{®}, Gluadin^{®}, Hostapon^{®} KCG oder Amisoft^{®} seit langem im Handel erhältlich.
- Alkyl- und/oder Alkenyl-Oligoglykosidcarboxylate, -sulfate, -phosphate und/oder -isethionate,
- Acyllactylate und
- Hydroxymischethersulfate.

Sofern die milden anionischen Tenside Polyglycoletherketten enthalten, ist es ganz besonders bevorzugt, dass diese eine eingeengte Homologenverteilung aufweisen. Weiterhin ist es im Falle von milden anionischen Tensiden mit Polyglycolethereinheiten bevorzugt, dass die Zahl der Glykolethergruppen 1 bis 20 beträgt, bevorzugt 2 bis 15, besonders bevorzugt 2 bis 12. Besonders milde anionische Tenside mit Polyglykolethergruppen ohne eingeschränkte Homologenverteilung können beispielsweise auch erhalten werden, wenn einerseits die Zahl der Polyglykolethergruppen 4 bis 12 beträgt und als Gegenion Zn- oder Mg-ionen gewählt werden. Ein Beispiel hierfür ist das Handelsprodukt Texapon^{®} ASV.

Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden (Tampho) werden solche oberflächenaktiven Verbindungen verstanden, die zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside (Tnio) sind beispielsweise
- Anlagerungsprodukte von 2 bis 50 Mol Ethylenoxid und/oder 0 bis 5 Mol Propylenoxid an lineare und verzweigte Fettalkohole mit 6 bis 30 C-Atomen, die Fettalkoholpolyglykolether bzw. die Fettalkoholpolypropylenglykolether bzw. gemischte Fettalkoholpolyether,
- Anlagerungsprodukte von 2 bis 50 Mol Ethylenoxid und/oder 0 bis 5 Mol Propylenoxid an lineare und verzweigte Fettsäuren mit 6 bis 30 C-Atomen, die Fettsäurepolyglykolether bzw. die Fettsäurepolypropylenglykolether bzw. gemischte Fettsäurepolyether,
- Anlagerungsprodukte von 2 bis 50 Mol Ethylenoxid und/oder 0 bis 5 Mol Propylenoxid an lineare und verzweigte Alkylphenole mit 8 bis 15 C-Atomen in der Alkylgruppe, die Alkylphenolpolyglykolether bzw. die Alkylpolypropylenglykolether, bzw. gemischte Alyklphenolpolyether,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol^{®} - Typen (Cognis),
- alkoxilierte Triglyceride,
- alkoxilierte Fettsäurealkylester der Formel (Tnio-1)

   R¹CO-(OCH₂CHR²)_{w}OR3 (Tnio-1)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, R¹O[CH₂CH(CH₃)O]ₓ(CH₂CHR²O)_{y}[CH₂CH(OH)R³]_{z} mit R¹ stehend für einen linearen oder verzweigten, gesättigten oder ungesättigten Alkyl- und/oder Alkenylrest mit 2 bis 30 C - Atomen, R² stehend für Wasserstoff, einen Methyl-, Ethyl-, Propyl- oder isoPropylrest, R³ stehend für einen linearen oder verzweigten Alkylrest mit 2 bis 30 C - Atomen, x stehend für 0 oder eine Zahl von 1 bis 20, Y für eine Zahl von 1 bis 30 und z stehend für die Zahl 1, 2, 3 , 4 oder 5.
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside,
- Zuckertenside vom Typ der Fettsäure-N-alkylpolyhydroxyalkylamide,
- Fettsäureamidpolyglycolether, Fettaminpolyglycolether,
- Mischether bzw. Mischformale und Polysorbate.

Kationische Tenside der Formel (Tkat1-1) können zusätzlich verwendet werden.

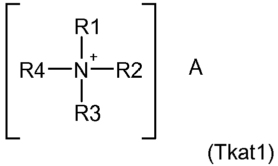

In der Formel (Tkat1) stehen R1, R2, R3 und R4 für jeweils unabhängig voneinander für Wasserstoff, eine Methylgruppe, eine Phenylgruppe, eine Benzylgruppe, für einen gesättigten, verzweigten oder unverzweigten Alkylrest mit einer Kettenlänge von 8 bis 30 Kohlenstoffatomen, welcher gegebenenfalls mit einer oder mehreren Hydroxygruppen substituiert sein kann. A steht für ein physiologisch verträgliches Anion, beispielsweise Halogenide wie Chlorid oder Bromid sowie Methosulfate.

Beispiele für Verbindungen der Formel (Tkat1) sind Lauryltrimehtylammoniumchlorid, Cetyltrimethylammoniumchlorid, Cetyltrimethylammoniumbromid, Cetyltrimethylammoniummethosulfat, Dicetyldimethylammoniumchlorid, Tricetylmethylammoniumchlorid, Stearyltrimethylammoniumchlorid, Distearyldimethylammoniumchlorid, Lauryldimethylbenzylammoniumchlorid, Behenyltrimethylammoniumchlorid, Behenyltrimethylammoniumbromid, Behenyltrimethylammoniummethosulfat.

Die Tenside (T) werden in Mengen von 0,05 - 45 Gew.%, bevorzugt 0,1 - 30 Gew.% und ganz besonders bevorzugt von 0,5 - 25 Gew.%, bezogen auf das gesamte erfindungsgemäß verwendete Mittel, eingesetzt.

Erfindungsgemäß verwendbare Emulgatoren sind beispielsweise
- Anlagerungsprodukte von 4 bis 30 Mol Ethylenoxid und/oder 0 bis 5 Mol Pro pylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine, sowohl aus tierischem Gewebe (Zoosterine, Cholesterin, Lanosterin) wie aus pflanzlichen Fetten (Phytosterine, Ergosterin, Stigmasterin, Sitosterin) oder aus Pilzen und Hefen (Mykosterine),
- Phospholipide (Lecithine, Phopshatidylcholine),
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH).

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Mit besonderem Vorzug enthalten die erfindungsgemäßen Zusammensetzungen Fettstoffe (Fat) als weiteren Wirkstoff. Unter Fettstoffen (Fat) sind zu verstehen Fettsäuren, Fettalkohole, natürliche und synthetische Wachse, welche sowohl in fester Form als auch flüssig in wäßriger Dispersion vorliegen können, und natürliche und synthetische kosmetische Ölkomponenten zu verstehen.

Als Fettsäuren (Fatac) können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. Bevorzugt beträgt die Menge 0,5 - 10 Gew.%, wobei ganz besonders vorteilhaft Mengen von 1 - 5 Gew.% sein können.

Als Fettalkohole (Fatal) können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden. Die Fettalkohole werden in Mengen von 0,1 - 30 Gew.-%, bezogen auf die gesamte Zubereitung, bevorzugt in Mengen von 0,1 - 20 Gew.-% eingesetzt.

Als natürliche oder synthetische Wachse (Fatwax) können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Die Gesamtmenge an Öl- und Fettkomponenten in den erfindungsgemäßen Mitteln beträgt üblicherweise 0,5 - 75 Gew.-%, bezogen auf das gesamte Mittel. Mengen von 0,5 - 35 Gew.-% sind erfindungsgemäß bevorzugt.

Eine weitere bevorzugte Gruppe von Inhaltsstoffen der erfindungsgemäßen Zusammensetzungen mit dem erfindungsgemäßen Wirkstoffkomplex sind Vitamine, Provitamine oder Vitaminvorstufen. Vitamine, Pro-Vitamine und Vitaminvorstufen sind dabei besonders bevorzugt, die den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.:
Vitamin B₁ (Thiamin)
Vitamin B₂ (Riboflavin)
Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Pantothensäure wird bevorzugt als Derivat in Form der stabileren Calciumsalze und Natriumsalze (Ca-Pantothenat, Na-Pantothenat) in der vorliegenden Erfindung eingesetzt.
Vitamin B₆ (Pyridoxin sowie Pyridoxamin und Pyridoxal).
   Die genannten Verbindungen des Vitamin B -Typs insbesondere Vitamin B₃, B₅ und B₆, sind in den erfindungsgemäßen Mitteln bevorzugt in Mengen von 0,05 - 10 Gew.-%, bezogen auf das gesamte Mittel, enthalten. Mengen von 0,1-5 Gew.-% sind besonders bevorzugt.
Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.
Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.
Vitamin F. Unter dem Begriff "Vitamin F" werden üblicherweise essentielle Fettsäuren, insbesondere Linolsäure, Linolensäure und Arachidonsäure, verstanden.
Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäßen Zusammensetzungen Vitamine, Provitamine und Vitaminvorstufen aus den Gruppen A, B, E und H. Panthenol, Pantolacton, Pyridoxin und seine Derivate sowie Nicotinsäureamid und Biotin sind besonders bevorzugt.

Eine besonders bevorzugte Gruppe von Inhaltsstoffen in den erfindungsgemäßen kosmetischen Zusammensetzungen sind die im folgenden genannten Betaine: Carnitin, Carnitintartrat, Carnitin Magnesiumcitrat, Acetylcarnitin, Betalaine, 1,1-Dimethyl-Prolin, Cholin, Cholinchlorid, Cholinbitartrat, Cholindihydrogencitrat und die in der Literatur als Betain bezeichnete Verbindung N,N,N-trimethylglycin.

Bevorzugt werden Carnitin, Histidin, Cholin sowie Betain verwendet. In einer besonders bevorzugten Ausführungsform der Erfindung wird als Wirkstoff L-Carnitintartrat eingesetzt.

In einer weiteren erfindungsgemäß bevorzugten Ausführungsform enthalten die erfindungsgemäßen Zusammensetzungen Biochinone. In den erfindungsgemäßen Mitteln sind unter geeigneten Biochinonen ein oder mehrere Ubichinon(e) und/oder Plastochinon(e) zu verstehen. Die erfindungsgemäß bevorzugten Ubichinone weisen die folgende Formel auf: 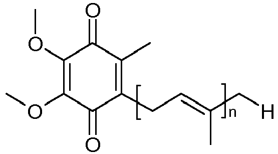 mit n = 6, 7, 8, 9 oder 10.

Das Coenzym Q-10 ist hierbei am bevorzugtesten.

Bevorzugte erfindungsgemäße Zusammensetzungen enthalten Purin und/oder Purinderivate in engeren Mengenbereichen. Hier sind erfindungsgemäß bevorzugte kosmetische Mittel dadurch gekennzeichnet, dass sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten. Erfindungsgemäß bevorzugte kosmetische Mittel sind dadurch gekennzeichnet, dass sie Purin, Adenin, Guanin, Harnsäure, Hypoxanthin, 6-Purinthiol, 6-Thioguanin, Xanthin, Coffein, Theobromin oder Theophyllin enthalten. In haarkosmetischen Zubereitungen ist Coffein am bevorzugtesten.

In einer weiteren bevorzugten Ausführungsform der vorliegenden Erfindung enthält das kosmetische Mittel Ectoin ((*S*)-2-Methyl-1,4,5,6-tetrahydro-4-pyrimidincarbonsäure. Erfindungsgemäß besonders bevorzugt sind Mittel, die - bezogen auf ihr Gewicht - 0,00001 bis 10,0 Gew.-%, vorzugsweise 0,0001 bis 5,0 Gew.-% und insbesondere 0,001 bis 3 Gew.-% der Wirkstoffe aus der Gruppe, die gebildet wird von Carnitin, Coenzym Q-10, Ectoin, ein Vitamin der B - Reihe, einem Purin und deren Derivaten oder physiologisch vertretbaren Salze enthalten.

Ein ganz besonders bevorzugter Pflegezusatz in den erfindungsgemäßen Haarbehandlungsmitteln ist Taurin. Unter Taurin wird ausschließlich 2-Aminoethansulfonsäure und unter einem Derivat die explizit genannten Derivate des Taurines verstanden. Unter den Derivaten des Taurines werden N-Monomethyltaurin, N,N-Dimethyltaurin, Taurinlysylat, Taurintartrat, Taurinornithat, Lysyltaurin und Ornithyltaurin verstanden.

Besonders bevorzugt sind erfindungsgemäße Mittel, die - bezogen auf ihr Gewicht - 0,0001 bis 10,0 Gew.-%, vorzugsweise 0,0005 bis 5,0 Gew.-%, besonders bevorzugt 0,001 bis 2,0 Gew.-% und insbesondere 0,001 bis 1,0 Gew.-% Taurin und/oder eines Derivates des Taurines enthalten. Die Wirkung der erfindungsgemäßen Zusammensetzungen kann weiterhin durch eine 2-Pyrrolidinon-5-carbonsäure und deren Derivate (J) gesteigert werden. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen 0,05 bis 10 Gew.%, bezogen auf das gesamte Mittel, besonders bevorzugt 0,1 bis 5, und insbesondere 0,1 bis 3 Gew.%.

Durch die Verwendung von Pflanzenextrakten als Pflegestoffe können die erfindungsgemäßen Haarbehandlungsmittel besonders naturnah und dennoch sehr effektiv in ihrer Pflegeleistung formuliert werden. Gegebenenfalls kann dabei sogar auf ansonsten übliche Konservierungsmittel verzichtet werden. Erfindungsgemäß sind vor allem die Extrakte aus Grünem Tee, Eichenrinde,

Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Baldrian, Wiesenschaumkraut, Quendel, Schafgarbe, Thymian, Melisse, Hauhechel, Huflattich, Eibisch, Meristem, Ginseng, Kaffee, Kakao, Moringa, Ingwerwurzel und ayurvedische Pflanzenextrakte wie beispielsweise Aegle Marmelos (Bilwa), Cyperus Rotundus (Nagar Motha), Emblica Officinalis (Amalki), Morida Citrifolia (Ashyuka), Tinospora Cordifolia (Guduchi), Santalum album, (Chandana), Crocus Sativus (Kumkuma), Cinnamonum Zeylanicum und Nelumbo Nucifera (Kamala), Süßgräser wie Weizen, Gerste, Roggen, Hafer, Dinkel, Mais, die verschiedenen Sorten der Hirse (Rispenhirse, Fingerhirse, Kolbenhirse als Beispiele), Zuckerrohr, Weidelgras, Wiesenfuchsschwanz, Glatthafer, Straußgras, Wiesenschwingel, Pfeifengras, Bambus, Baumwollgras, Lampenputzergräser, Andropogonodeae (Imperata Cylindrica auch Flammengras oder Cogon Gras genannt), Büffelgras, Schlickgräser, Hundszahngräser, Liebesgräser, Cymbopogon (Zitronengras), Oryzeae (Reis), Zizania (Wildreis), Strandhafer, Staudenhafer, Honiggräser, Zittergräser, Rispengräser, Quecken und Echinacea, insbesondere Echinacea purpurea (L.) Moench, aller Arten von Wein sowie Perikarp von Litchie chinensis bevorzugt.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Gelegentlich kann es erforderlich sein anionische Polymere zu verwenden. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfon-säure, das beispielsweise unter der Bezeichnung Rheothik^{®}11-80 im Handel erhältlich ist.

Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein solches Polymer ist in dem Handelsprodukt Sepigel^{®}305 der Firma SEPPIC enthalten.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Die anionischen Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Die erfindungsgemäßen Mittel können in einer weiteren Ausführungsform nichtionogene Polymere enthalten.

Geeignete nichtionogene Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) und Natrosol^{®}-Typen (Hercules) vertrieben werden.
- Stärke und deren Derivate, insbesondere Stärkeether, beispielsweise Structure^{®} XL (National Starch), eine multifunktionelle, salztolerante Stärke;
- Schellack
- Polyvinylpyrrolidone, wie sie beispielsweise unter der Bezeichnung Luviskol^{®} (BASF) vertrieben werden.

Die nichtionischen Polymere sind in den erfindungsgemäßen Zusammensetzungen bevorzugt in Mengen von 0,05 bis 10 Gew.-%, bezogen auf das gesamte Mittel, enthalten. Mengen von 0,1 bis 5 Gew.-% sind besonders bevorzugt.

In einer weiteren Ausführungsform sollten die erfindungsgemäßen Mittel zusätzlich mindestens einen UV-Lichtschutzfilter enthalten. UVB-Filter können öllöslich oder wasserlöslich sein.

Als öllösliche Substanzen sind z.B. zu nennen:
- 3-Benzylidencampher, z.B. 3-(4-Methylbenzyliden)campher;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3-phenyl-zimtsäure-2-ethylhexylester (Octocrylene);
- Ester der Salicylsäure, vorzugsweise Salicylsäure-2-ethylhexylester, Salicylsäure-4-isopropylben-zylester, Salicylsäurehomomenthylester;
- Derivate des Benzophenons, vorzugsweise 2-Hydroxy-4-methoxybenzophenon, 2-Hydroxy-4-meth-oxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon.
- Propan-1,3-dione, wie z.B. 1-(4-tert.Butylphenyl)-3-(4'methoxyphenyl)propan-1,3-dion; Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sul-fonsäure und ihre Salze;
- Sulfonsäurederivate des 3-Benzylidencamphers, wie z.B. 4-(2-Oxo-3-bornylidenmethyl)benzolsul-fonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Pigmente, insbesondere feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen.

Ferner können die kosmetischen Mittel weitere Wirk-, Hilfs- und Zusatzstoffe enthalten, wie beispielsweise
- Strukturanten wie Maleinsäure und Milchsäure,
- Quellmittel wie Harnstoff, Allantoin, Carbonate oder Hydantoin,
- Dimethylisosorbid und Cyclodextrine,
- Farbstoffe zum Anfärben des Mittels,
- Antischuppenwirkstoffe wie Piroctone Olamine, Zink Omadine und Climbazol,
- Komplexbildner wie EDTA, NTA, β-Alanindiessigsäure und Phosphonsäuren,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien,
- Parfümöle, Duftstoffe und Riechstoffe.

Bezüglich weiterer fakultativer Komponenten sowie die eingesetzten Mengen dieser Komponenten wird ausdrücklich auf die dem Fachmann bekannten einschlägigen Handbücher verwiesen.

Ein weiterer Erfindungsgegenstand ist daher ein Verfahren zur Haarbehandlung, in dem ein Haarbehandlungsmittel gemäß Anspruch 1 auf das Haar aufgetragen wird und nach einer Einwirkungszeit vom Haar gespült wird.

Die Einwirkungszeit beträgt bevorzugt wenige Sekunden bis 100 Minuten, besonders bevorzugt 1 bis 50 Minuten und ganz besonders bevorzugt 1 bis 30 Minuten.

Erfindungsgemäß ist weiterhin ein Verfahren, bei welchem ein kosmetisches Mittel gemäß Anspruch 1 auf das Haar aufgetragen wird und dort verbleibt. Unter "auf dem Haar verbleiben" wird erfindungsgemäß verstanden, dass das Mittel nicht unmittelbar nach dessen Anwendung wieder aus dem Haar ausgespült wird. Vielmehr verbleibt das Mittel in diesem Falle mehr als 100 Minuten bis hin zur nächsten Haarwäsche auf dem Haar.

Die folgenden Beispiele sollen den Gegenstand der vorliegenden Erfindung erläutern ohne ihn jedoch zu beschränken.

### Beispiele

Alle Mengenangaben sind, soweit nicht anders vermerkt, Gewichtsteile. Die folgenden Rezepturen wurden unter Anwendung bekannter Herstellungsverfahren bereitgestellt

| | E1 | E2 | E3 | E4 |
|---|---|---|---|---|
| Cetearyl Alcohol | 4,5 | 6,0 | 4,0 | 3,0 |
| Dicaprylyl Carbonate | 0,5 | 1,0 | 0,3 | --- |
| PEG-30 Dipolyhydroxystearate | 0,7 | 1,0 | 1,5 | 2,0 |
| Distearoyl Hydroxyethylmonium Methosulfate | 2,0 | 2,0 | 0,5 | 0,5 |
| Behentrimonium Chloride | 2,0 | 2,5 | 3,0 | 0,2 |
| Polyquaternium-67 | 0,1 | 0,15 | 0,2 | --- |
| Dimethicone, 60.000 cSt | 1,0 | 1,5 | 1,0 | 1,5 |
| Panthenol | 0,2 | 0,2 | 0,2 | 0,2 |
| Glycerin | 0,7 | 0,7 | 0,2 | 0,2 |
| Parfum | 0,6 | 0,7 | --- | --- |
| Isopropylmyristat | --- | 0,2 | --- | 1,0 |
| Quaternium-87 | --- | --- | --- | 1,5 |
| Behenoyl PG-Trimonium Chloride | --- | --- | --- | 2,0 |
| Propylene Glycole Dibenzoate | 0,1 | 0,5 | 0,25 | 1,0 |
| Proteinhydrolysat (Nutrilan I, oder Gluadin W40, oder Nutrilan Keratin, oder ProSina® einzeln oder in Mischungen) | 0,5 | 0,7 | 0,1 | 0,2 |
| Niacinamide | 0,1 | 0,2 | --- | --- |
| pH - Wert Einstellung mit Citronensäure, Weinsäure und deren Salzen und ggf. NaOH oder KOH) bis auf: | pH 2,5 | pH 2,5 | pH 4,0 | pH 4,0 |
| Konservierungsmittel( Phenaoxyethanol, Parabene, Sorbat und deren Mischungen) | 0,4 | 0,4 | 0,5 | 0,5 |
| Wasser | Ad 100 | Ad 100 | Ad 100 | Ad 100 |

Alle Zusammensetzungen wurden auf ihre Leitfähigkeit hin untersucht. Die Leitfähigkeit von W/O - Emulsionen ist erheblich niedriger und geht gegen Null im Vergleich zu O/W - Emulsionen. Alle erfindungsgemäßen Emulsionen haben sich nach dieser Methode als W/O - Emulsionen erwiesen. Die vorstehenden Zusammensetzungen weisen eine Viskosität zwischen 600 und 40000 mPas gemessen nach Brookfield bei 25 °C und mit Helipath auf. Alle Zusammensetzungen können in einer üblichen Schüttflasche oder in Tiegeln abgefüllt werden. Sie können aber auch in einer entsprechenden Pumpverpackung mit einer üblichen Dosierpumpe versehen sein und verpackt werden. Die Dosierpumpe kann dabei selbstverständlich ein Sieb enthalten, so dass über die Zumischung von Luft im Pumpenkopf die Zusammensetzungen als Schaum ausgebracht werden. Schließlich ist die Abfüllung in Aerosoldosen zusammen mit üblichen Treibmitteln wie iso-Propan, Propan, iso-Butan, Butan, tert.-Butan, Pentan, iso-Pentan, ter.-Pentan, Neopentan oder Dimethylether sowie deren beliebige Mischungen im Verhältnis von 90 : 10, bevorzugt 92:8 (Treibmittel) möglich. Auch in diesem Falle wird ein besonders cremiger und stabiler Schaum erhalten, welcher sich sehr gut im Haar verteilen lässt.

Die anwendungstechnische Beurteilung erfolgte durch 5 Friseure an jeweils 20 Köpfen im sogenannten Halbseitentest. Geprüft wurden die Rezepturen E1 bis E4 gegen die Rezepturen V1 bis V4. Die Rezepturen V1 bis V4 sind die Rezepturen E1 bis E4 jedoch ohne Proteinhydrolysat und PEG-30 Dipolyhydroxystearate, die erfindungsgemäße Wirkstoffkombination.

Die Haare wurden wie üblich mit einem Shampoo gewaschen und mit einem Handtuch getrocknet. Die Kopfhälften wurden mit einem Kamm in der Mitte gescheitelt und auf jede Kopfhälfte wurden 5 g der jeweiligen Prüfzusammensetzung aufgetragen, also E1 gegen V1 usw. Nach einer Einwirkzeit von 5 Minuten bei etwa 25°C wurden die Zusammensetzungen unter fließendem Wasser von etwa 32 °C für 3 Minuten wieder ausgespült. Anschließend erfolgte die vergleichende Beurteilung durch die 5 Friseure.

Die erfindungsgemäßen Zusammensetzungen E1 bis E4 erwiesen sich in den Parametern Bearbeitung des nassen Haares mit dem Föhn, Kämmbarkeit, Griff des nassen sowie des trockenen Haares, Gefühl der nassen und trockenen Haarspitzen, Body des trockenen Haares, sowie Glanz des trockenen Haares jeweils um mindestens 0,67 Noten besser.

Weiterhin wurde die Langzeitwirkung der erhaltenen Frisur überprüft. Dazu wurden die Modelle 24 h nach der Anwendung nochmals beurteilt. Hier erwiesen sich alle erfindungsgemäßen Zusammensetzungen in jedem Parameter um mindestens 1 Note besser.

## Patentansprüche

1. Verfahren zur Behandlung keratinischer Fasern **dadurch gekennzeichnet, dass** eine kosmetische Zusammensetzung zur Pflege keratinischer Fasern, enthaltend - jeweils bezogen auf die gesamte Zusammensetzung -
a) PEG-30 Dipolyhydroxydistearate in einer Gesamtmenge von 0,01 bis 5,0 Gew.%,
b) mindestens ein Proteinhydrolysat sowie Mischungen von Proteinhydrolysaten in einer Gesamtmenge von 0,01 bis 10,0 Gew.% und
c) in Form einer W/O - Emulsion mit mindestens 50 Gew.% Wasser
auf die keratinischen Fasern aufgetragen wird und nach einer Einwirkzeit von wenigen Sekunden bis hin zu 45 Minuten wieder ausgespült wird..

2. Verfahren zur Behandlung keratinischer Fasern nach Anspruch 1, **dadurch gekennzeichnet, dass** die kosmetische Zusammensetzung weiterhin mindestens eine quartäre Ammoniumverbindung enthält in einer Gesamtmenge von 0,1 bis 10,0 Gew.% bezogen auf das Gewicht der gesamten Zusammensetzung, ausgewählt aus einer der Gruppen
i) der Esterquats und/oder
ii) der quarternären Imidazoline der Formel (Tkat2), 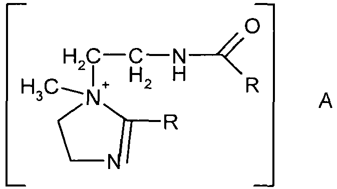 in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder
iii) der Amine und/oder kationisierten Amine und/oder
iv) Poly(methacryloyloxyethyltrimethylammoniumverbindungen) und/oder;
v)quaternisierten Cellulose-Derivaten, insbesondere Polyquaternium 10, Polyquaternium-24, Polyquaternium-27, Polyquaternium-67, Polyquaternium-72, und/oder
vi)kationische Alkylpolyglycoside und/oder
vii)kationisiertem Honig und/oder
viii)kationischen Guar-Derivaten und/oder
ix)Chitosan und/oder
x)polymeren Dimethyldiallylammoniumsalzen und deren Copolymeren mit Estern und Amiden von Acrylsäure und Methacrylsäure, insbesondere Polyquaternium-7 und/oder
xi)Copolymeren des Vinylpyrrolidons mit quaternierten Derivaten des Dialkylaminoalkylacrylats und -methacrylats, insbesondere Polyquaternium-11 und/oder
xii)Vinylpyrrolidon-Vinylimidazoliummethochlorid-Copolymeren, insbesondere Polyquaternium-16 und/oder
xiii)quaterniertem Polyvinylalkohol und/oder
xiv) Polyquaternium-74,
xv) Kationische alkyloligoglucoside und/oder
xvi) Polyquaternium-71
sowie deren Mischungen.

3. Verfahren zur Behandlung keratinischer Fasern nach einem der Ansprüche 1 oder 2, **dadurch gekennzeichnet, daß** das Proteinhydrolysat ausgewählt ist aus den Hydrolysaten auf der Basis von Elastin-, Kollagen-, Keratin-, Seiden- und Milcheiweiß, Soja-, Mandel-, Erbsen-, Kartoffel- und Weizen, Fischen, Algen oder Perlen sowie deren Mischungen.

4. Verfahren zur Behandlung keratinischer Fasern nach Anspruch 3, **dadurch gekennzeichnet, dass** das Proteinhydrolysat ausgewählt ist aus den Hydrolysaten von Kollagen, Seide oder Keratin sowie deren Mischungen.

5. Verfahren zur Behandlung keratinischer Fasern nach einem der Ansprüche 1 bis 4, **dadurch gekennzeichnet, dass** zusätzlich mindestens ein Wirkstoff ausgewählt aus der Gruppe, die gebildet wird von Carnitin, Taurin, Coenzym Q-10, Ectoin, einem Purin und dessen Derivaten, insbesondere Coffein, oder deren physiologisch verträglichen Salze, sowie einem Vitamin der B - Reihe, insbesondere Panthenol, enthalten ist.

6. Verfahren zur Behandlung keratinischer Fasern nach einem der Ansprüche 1 bis 5, **dadurch gekennzeichnet, dass** weiterhin Esteröle in einer Gesamtmenge von 0,01 bis 20 Gew.%, bevorzugt 0,01 bis 10,0 Gew.%, besonders bevorzugt 0,01 bis 7,5 Gew.%, höchst bevorzugt von 0,1 bis 5,0 Gew.% ausgewählt aus Amaranthsamenöl, Aprikosenkernöl, Arganöl, Marulaöl, Macadamiaöl, Mandelöl, insbesondere Süßmandelöl, Sesamöl, Sonnenblumenöl, Olivenöl, Orangenöl, Kapkastanienöl, Weizenkeimöl, Pfirsichkernöl, Isopropylmyristate, Dicaprylylcarbonate und/oder Coco-caprylate sowie deren Mischungen, enthalten ist.

7. Verfahren zur Behandlung keratinischer Fasern nach einem der Ansprüche 1 bis 7, **dadurch gekennzeichnet, dass** die Zusammensetzung eine W/O - Cremehaarkur mit einer Viskosität von 600 bis 40000 mPas, gemessen nach Brookfield mit Helipath bei einer Temperatur von 25 °C ist.

## Claims

1. A method for treating keratin fibers, **characterized in that** a cosmetic composition for nourishing keratin fibers, containing, in each case based on the total composition,
(a) PEG-30 dipolyhydroxydistearate in a total amount of 0,01 to 5.0 wt.%,
(b) at least one protein hydrolyzate and mixtures of protein hydrolyzates in a total amount of 0.01 to 10.0 wt.%, and
(c) in the form of a W/O emulsion containing at least 50 wt.% water,
is applied to the keratin fibers and rinsed out again after a reaction time of a few seconds up to 45 minutes.

2. The method for treating keratin fibers according to claim 1, **characterized in that** the cosmetic composition further contains at least one quaternary ammonium compound in a total amount of 0.1 to 10.0 wt.%, based on the weight of the total composition, selected from one of the groups consisting of
i) esterquats, and/or
ii) quaternary imidazolines of formula (Tkat2), 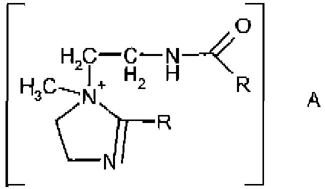 in which the functional groups R, independently of one another, each represent a saturated or unsaturated, linear or branched hydrocarbon functional group having a chain length of 8 to 30 carbon atoms and A represents a physiologically acceptable anion, and/or
iii) amines and/or cationized amines, and/or
iv) poly(methacryloyloxyethyltrimethylammonium) compounds, and/or
v) quaternized cellulose derivatives, in particular Polyquaternium-10, Polyquaternium-24, Polyquaternium-27, Polyquaternium-67, Polyquaternium-72, and/or
vi) cationic alkyl polyglycosides, and/or
vii) cationized honey, and/or
viii) cationic guar derivatives, and/or
ix) chitosan, and/or
x) polymeric dimethyldiallylammonium salts and copolymers thereof with esters and amides of acrylic acid and methacrylic acid, in particular Polyquaternium-7, and/or
xi) copolymers of vinylpyrrolidone with quaternized derivatives of dialkylaminoalkyl acrylate and methacrylate, in particular Polyquaternium-11, and/or
xii) vinylpyrrolidone-vinylimidazolium methochloride copolymers, in particular Polyquaternium-16, and/or
xiii) quaternized polyvinyl alcohol, and/or
xiv) Polyquaternium-74,
xv) cationic alkyl oligoglucosides, and/or
xvi) Polyquaternium-71,
and mixtures thereof.

3. The method for treating keratin fibers according to one of claims 1 or 2, **characterized in that** the protein hydrolyzate is selected from the hydrolyzates based on elastin, collagen, keratin, silk and milk protein, soy, almond, pea, potato and wheat, fish, algae or pearls, and mixtures thereof.

4. The method for treating keratin fibers according to claim 3, **characterized in that** the protein hydrolyzate is selected from the hydrolyzates of collagen, silk or keratin, and mixtures thereof.

5. The method for treating keratin fibers according to one of claims 1 to 4, **characterized in that** at least one active ingredient is additionally contained, selected from the group formed by carnitine, taurine, coenzyme Q-10, ectoine, a purine and derivatives thereof, in particular caffeine, or physiologically acceptable salts thereof, and a vitamin of the B series, in particular panthenol.

6. The method for treating keratin fibers according to one of claims 1 to 5, **characterized in that** ester oils are further contained in a total amount of 0.01 to 20 wt.%, preferably 0.01 to 10.0 wt.%, particularly preferably 0.01 to 7.5 wt.%, most preferably 0.1 to 5.0 wt.%, selected from amaranth seed oil, apricot kernel oil, argan oil, marula oil, macadamia oil, almond oil, in particular sweet almond oil, sesame oil, sunflower oil, olive oil, orange oil, cape chestnut oil, wheat germ oil, peach kernel oil, isopropyl myristate, dicaprylyl carbonate and/or coco-caprylate, and mixtures thereof.

7. The method for treating keratin fibers according to one of claims 1 to 7, **characterized in that** the composition is a W/O cream hair masque having a viscosity of 600 to 40,000 mPas, measured according to Brookfield using a helipath at a temperature of 25°C.

## Revendications

1. Procédé de traitement de fibres de kératine, **caractérisé en ce qu'**on applique sur les fibres de kératine une composition cosmétique de soin de fibres de kératine contenant, rapporté respectivement à la composition totale :
a) des dipolyhydroxydistéarates PEG-30 à hauteur totale de 0,01 à 5,0 % en poids,
b) au moins un hydrolysat de protéines, ainsi que des mélanges d'hydrolysats de protéines, à hauteur totale de 0,01 à 10,0 % en poids,
c) sous forme d'une émulsion eau dans l'huile à au moins 50% en poids d'eau, et
on rince après une durée d'action de quelques secondes à jusqu'à 45 minutes.

2. Procédé de traitement de fibres de kératine selon la revendication 1, **caractérisé en ce que** la composition cosmétique contient en outre un composé ammonium quaternaire à hauteur de 0,01 à 10,0 % en poids rapporté au poids de la composition totale, choisi dans le groupe :
i) des esterquats, et/ou
ii) des imidazolines quaternaires de formule (Tkat2) : 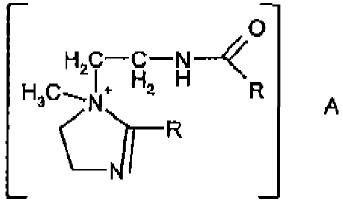 où les résidus R représentent, indépendamment les uns des autres, un résidu hydrocarbure saturé ou insaturé, linéaire ou ramifié, avec une chaine carbonée de 8 à 30 atome de carbone, et A représente un anion physiologiquement compatible, et/ou
iii) des amines et/ou amines quaternisées, et/ou
iv) des composés polymethacryloyloxyéthyltriméthylamnnonium, et/ou
v) des dérivés de cellulose quaternisés, en particulier Polyquaternium 10, Polyquaternium 24, Polyquaternium 27, Polyquaternium 67, Polyquaternium 72, et/ou
vi) des alkylpolyglycosides cationiques, et/ou
vii) du miel cationisé, et/ou
viii) des dérivés de guar cationisés, et/ou
ix) du chitosan, et/ou
x) des sels polymères de diméthyldiallylammonium et leurs copolymères avec des esters et des amides d'acide acrylique et d'acide méthacrylique, en particulier Polyquaternium 7, et/ou
xi) des copolymères de vinylpyrrolidone avec des dérivés quaternaires d'acrylate et de méthacrylate de dialkylaminoalkyle, en particulier Polyquaternium 11, et/ou
xii) des copolymères de vinylpyrrolidone et méthochlorure de vinylimidazolium, en particulier Polyquaternium 16, et/ou
xiii) un alcool polyvinylique quaternisé, et/ou
xiv) Polyquaternium 74,
xv) Des alkyloligoglucosides cationiques, et/ou
xvi) Polyquaternium 71,
ainsi que leurs mélanges.

3. Procédé de traitement de fibres de kératine selon une des revendications 1 ou 2, **caractérisé en ce que** l'hydrolysat de protéines est choisi parmi les hydrolysats à base de protéines d'élastine, de collagène, de kératine, de soie et de lait, de soja, d'amande, de pois, de pomme de terre, de blé, de poissons, d'algues ou de perles, ainsi que leurs mélanges.

4. Procédé de traitement de fibres de kératine selon la revendication 3, **caractérisé en ce que** l'hydrolysat de protéines est choisi parmi les hydrolysats de collagène, soie ou kératine, ainsi que leurs mélanges.

5. Procédé de traitement de fibres de kératine selon une des revendications 1 à 4, **caractérisé en ce qu'**il contient en plus au moins un agent actif choisi dans le groupe constitué de la carnitine, la taurine, le coenzyme Q10, l'ectoine, une purine et ses dérivés, en particulier la caféine, ou leurs sels physiologiquement compatibles, ainsi qu'une vitamine de la série B, en particulier le panthénol.

6. Procédé de traitement de fibres de kératine selon une des revendications 1 à 5, **caractérisé en ce qu'**il contient en outre des huiles esters à hauteur de 0,01 à 20 % en poids, de préférence 0,01 à 10,0 % en poids, particulièrement préférentiellement 0,01 à 7,5 % en poids, idéalement 0,1 à 5,0 % en poids, choisis parmi l'huile de graine d'amaranthe, l'huile de noyau d'abricot, l'huile d'argan, l'huile de marula, l'huile de macadamia, l'huile d'amande, en particulier l'huile d'amande douce, l'huile de sésame, l'huile de tournesol, l'huile d'olive, l'huile d'orange, l'huile de yangu, l'huile de germe de blé, l'huile de noyau de pèche, le myristate d'isopropyle, le carbonate de dicaprylyle et/ou le coco-caprylate ainsi que leurs mélanges.

7. Procédé de traitement de fibres de kératine selon une des revendications 1 à 7, **caractérisé en ce que** la composition est une crème de soin capillaire eau dans l'huile avec une viscosité de 600 à 40 000 mPa.s mesurée selon Brookfield avec Helipath à une température de 25°C.
